# EUROPÄISCHE PATENTSCHRIFT

(11) **EP 3 593 701 B1**
(45) Veröffentlichungstag und Bekanntmachung des Hinweises auf die Patenterteilung: **15.04.2026**
(21) Anmeldenummer: 19180730.4
(22) Anmeldetag: 18.06.2019
(51) Int. Cl.: A61B 1/005, A61B 1/008, A61B 1/267

(54) **VERFORMBARER SPATEL FÜR EIN LARYNGOSKOP**
DEFORMABLE SPATULA FOR A LARYNGOSCOPE
SPATULE DÉFORMABLE POUR UN LARYNGOSCOPE

(30) Priorität: 12.07.2018 DE 102018116885
(43) Veröffentlichungstag der Anmeldung: 15.01.2020
(73) Patentinhaber: Karl Storz SE & Co. KG, 78532 Tuttlingen (DE)
(72) Erfinder: Attinger, Jürg, 8260 Stein am Rhein (CH)

(56) Entgegenhaltungen:
- DE-A1- 102016 113 498
- KR-A- 20080 056 779
- US-A1- 2010 261 968
- US-A1- 2013 310 650
- US-A1- 2015 031 957

## Beschreibung

Die vorliegende Erfindung ist auf einen verformbaren Spatel für ein adaptierbares oder adaptives Laryngoskop, insbesondere für ein adaptierbares oder adaptives Intubationslaryngoskop oder ein adaptierbares oder adaptives Laryngoskop für die Larynxchirurgie oder andere Aufgaben innerhalb der Hals-Nasen-Ohren-Heilkunde oder Oto-Rhino-Laryngologie bezogen.

Zur endotrachealen Intubation in Anästhesie, Notfallmedizin und Intensivmedizin sowie der Chirurgie des Kehlkopfs (Larynx) wird für eine Intubation oder für chirurgische Maßnahmen ein unverlegter Zugang zum Kehlkopf, den Stimmbändern und letztlich der Trachea benötigt. Dabei dient ein Laryngoskop dazu, die Zunge nach vorne bzw. rostral zu schieben. Ein Laryngoskop umfasst in der Regel einen mehr oder weniger gekrümmten Spatel, an dessen proximalem Ende in näherungsweise rechtem Winkel ein Griff angeordnet ist.

Um eine Anpassung an die Anatomie des Patienten zu ermöglichen, ist der Spatel in der Regel austauschbar. In einem Intubationsset befindet sich eine Vielzahl von Spateln unterschiedlicher Längen und unterschiedlicher Krümmungen. Für verschiedene Anwendungen und/oder unterschiedlicher Vorlieben des medizinischen Personals stehen ferner unterschiedliche Bauformen zur Verfügung, beispielsweise nach Macintosh, Miller, Dörges und McCoy, letzterer mit einem bewegbaren distalen Ende.

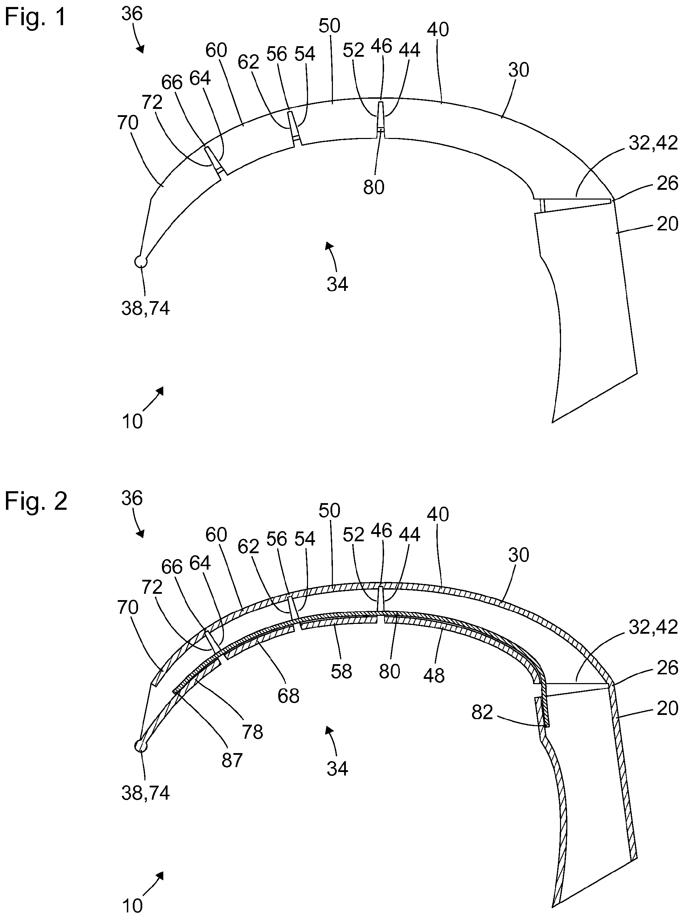

Ein Laryngoskop mit einem verformbaren distalen Ende ist auch in WO 97/30626 A1 beschrieben. Der Spatel 4 des Laryngoskops weist in einem mittleren Abschnitt 14 mehrere Schlitze 40 auf (Seite 6, Zeilen 24 bis 28; Figur 1). Die Schlitze 40 unterteilen den mittleren Abschnitt 14 in Segmente 42, die nur durch schmale, als Festkörpergelenke wirkende Stege miteinander verbunden sind (ebd.). Eine Schubstange 48 ist innerhalb eines Führungsrohrs 46 bewegbar (Seite 7, Zeilen 8, 9). Ein distales Ende der Schubstange 48 ist mit einem distalen Ende 20 des Spatels 4 verschweißt (Seite 7, Zeilen 5, 6, Figuren 1 bis 4). Ein als Schiebeeinrichtung 60 ausgebildetes Ende eines Betätigungshebels 56 an einem Griff 2 des Laryngoskops kann das proximale Ende 50 der Schubstange 48 nach distal schieben (Seite 7, Zeilen 13 bis 18), um den mittleren Abschnitt 14 des Spatels 4 zu krümmen (Seite 7, Zeilen 7 bis 10; Figur 2).

In FR 2 821 736 ist ein Laryngoskop 1 mit einem Griff 2 und einem Spatel 3 beschrieben (Seite 11 bis 14), Figuren 1 bis 3). Der Spatel 3 umfasst eine Mehrzahl gelenkig verbundener Segmente 14 (Seite 8, Zeilen 2 bis 7; Figur 1). Zur Formgebung des Spatels 3 ist ein flexibles Element 15 vorgesehen, dessen distales Ende 16 mit dem distalen Ende 9 des Spatels 3 verbunden ist, und dessen proximales Ende 17 relativ zu dem Griff 2 bewegbar ist (Seite 8, Zeilen 9 bis 14; Figur 1).

In US 2010/0261968 A1 ist ein bewegbares Laryngoskop 700 mit einem Griff 702 und einem Spatel 704 (Absatz [0074], Figur 7). Der Spatel 704 umfasst einen Hauptspatel 706, einen proximalen Spatel 708 und einen distalen Spatel 710, die durch Gelenke 712, 714 miteinander verbunden sind (Absatz [0075], Figur 7). Der Griff 702 umfasst eine Spatelsteuerkomponente 720 oder einen Knopf 902 zum Steuern der Krümmung des Spatels 704 (Absätze [0082], [0092]; Figuren 7, 9). Eine als proximaler Stößel ("proximal pusher") bezeichnete Einrichtung 1206 koppelt den Knopf 902 mit dem proximalen Spatel 708 an dem Gelenk 712 (Absätze [0102], [0104]; Figuren 13, 14). Eine als distaler Stößel ("distal pusher") bezeichnete Einrichtung 1208 koppelt einen Knopf 1402 mit dem distalen Spatel 710 an dem Gelenk 714 (ebd.).

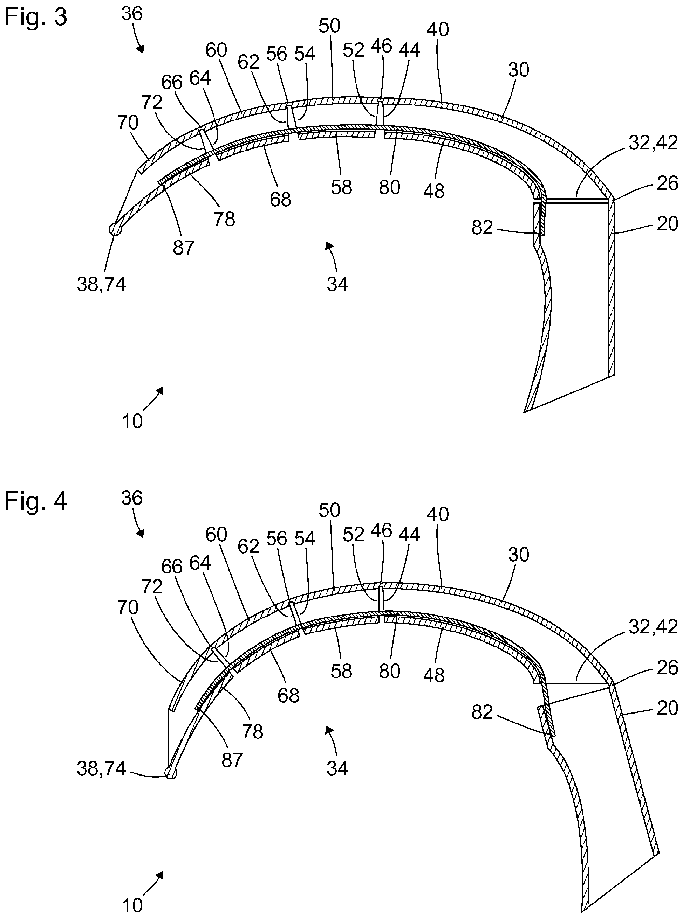

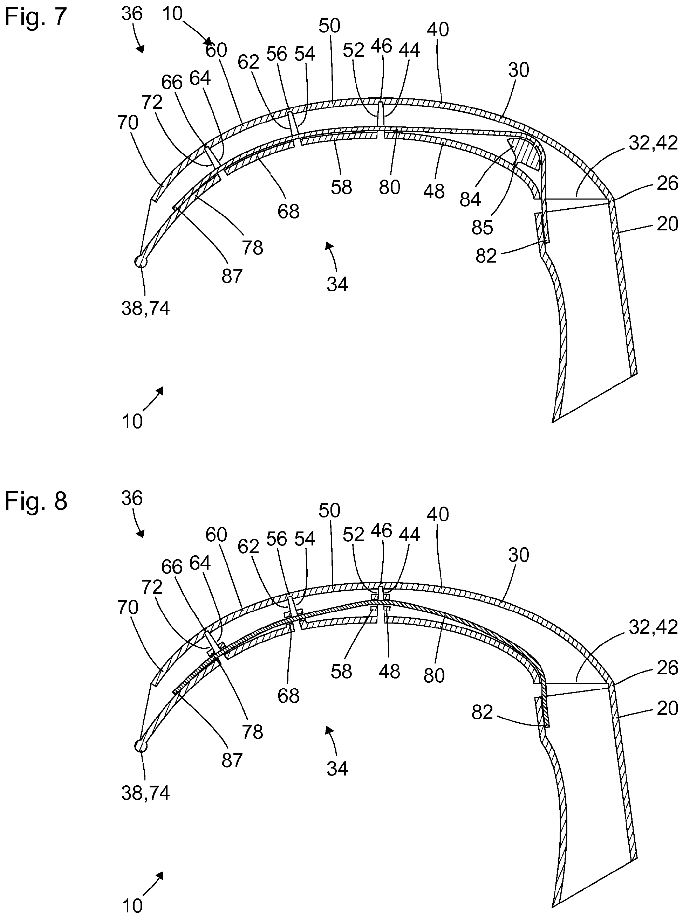

In WO 2011/150469 A1 ist ein Laryngoskop mit einem Spatel 1 und einem Griff 3 beschrieben. Das distale Ende des Spatels 1 wird durch mehrere Glieder 7A, 7B, 7C gebildet, die durch Gelenke 8A, 8B, 8C mit einander verbunden sind (Seite 17, viertletzter Absatz; Figuren 3, 11, 19, 20, 21, 22, 26). Die Krümmung des Spatels 1 kann mittels eines Bedienungselements 11 an dem Griff 3 verändert werden (Seite 17, drittletzter Absatz; Figuren 3, 11, 19, 20, 21, 22, 26). Das Bedienungselement 11 ist mittels Spanneinrichtungen D, M, P mit den Gliedern 7A, 7B, 7C verbunden (die Seiten 19, 20 überbrückender Absatz; Figuren 8, 9, 10, 22, 24).

In US 2011/0144436 A1 ist eine Vorrichtung 100, die einem Laryngoskop ähnelt und einen Griff 102 und einen Spatel 104 umfasst, beschrieben (Absatz [0027], Figur 1). Die Krümmung des Spatels 104 kann eingestellt, Teile des Spatels 104 oder der gesamte Spatel 104 können relativ zu dem Griff 102 eingestellt werden (Absatz [0028]).

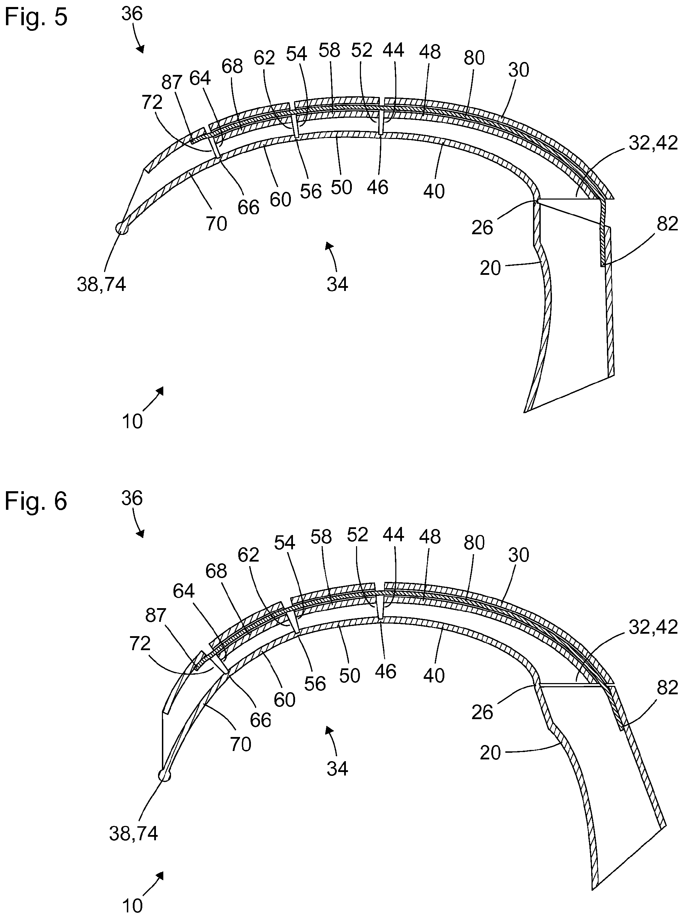

In US 2011/0319718 A1 ist ein Laryngoskop 100 mit einem Griff 104 und einem Spatel, der eine erste Armkomponente 108 und eine zweite Armkomponente 110 mit einem Gelenk 112 dazwischen umfasst, beschrieben (Absätze [0052], [0053], Figuren 6A, 6B). Eine Verbindungsstange oder eine Verbindungsschnur verbindet die zweite Armkomponente 110 derart mit dem Handgriff 104, dass ein Winkel zwischen der zweiten Armkomponente und dem Griff 104 unabhängig von der Winkelposition der ersten Armkomponente im Wesentlichen konstant ist (Absatz [0060], Figuren 6A, 6B).

In US 2015/031957 ist ein Laryngoskop mit Widerstandsrückkopplung beschrieben. Dieses Laryngoskop umfasst einen Schiebegriff, ein Triggerpad, eine Drehwelle, eine erste Übertragungsstange, eine zweite Übertragungsstange und eine zweistufige Hebelklinge. Der Schiebegriff hat eine gekrümmte Vorderseite, die mit dem zweistufig hebelnden Spatel verbunden ist. Die zweistufige Hebelklinge umfasst eine erste bewegliche Klinge, eine zweite bewegliche Klinge und eine feste Klinge. Die erste Übertragungsstange und die zweite Übertragungsstange sind
mit der Drehwelle verbunden, die an einem Antriebspfosten mit einem elastischen Element befestigt ist.

US 2010/261968 A1 offenbart den Oberbegriff von Anspruch 1. DE 10 2016 113498 A1 offenbart den Oberbegriff von Anspruch 9.

Eine Aufgabe der vorliegenden Erfindung besteht darin, einen verbesserten Spatel für ein adaptierbares oder adaptives Laryngoskop und ein verbessertes Laryngoskop zu schaffen. Diese Aufgabe wird durch die Gegenstände der unabhängigen Ansprüche gelöst. Weiterbildungen sind in den abhängigen Ansprüchen angegeben.

Ein Spatel für ein adaptierbares oder adaptives Laryngoskop umfasst ein proximales Ende, das mit einem Griffteil zum manuellen Halten des Laryngoskops gelenkig verbunden oder verbindbar ist, ein distales Ende zum Einführen in den Rachen eines Patienten, eine bei vorgesehener Verwendung des Spatels an der Zunge des Patienten anliegende erste Seite, eine bei vorgesehener Verwendung des Spatels dem Gaumen des Patienten zugewandte zweite Seite, eine Mehrzahl von in Längsrichtung des Spatels hintereinander angeordneten und starren Gliedern, die sich jeweils von der ersten Seite des Spatels bis zu der zweiten Seite des Spatels erstrecken, eine Mehrzahl von Gelenken, wobei jedes der Mehrzahl von Gelenken zwei benachbarte Glieder gelenkig verbindet, und eine Kraftübertragungseinrichtung zum Übertragen einer Kraft, mit einem proximalen Ende und einem distalen Ende, wobei das distale Ende der Kraftübertragungseinrichtung mit dem distalen Ende des Spatels mechanisch verbunden ist, wobei die Kraftübertragungseinrichtung zwischen ihrem distalen Ende und ihrem proximalen Ende relativ zu Gliedern des Spatels in Längsrichtung der Kraftübertragungseinrichtung bewegbar ist, wobei das proximale Ende der Kraftübertragungseinrichtung mit dem Griffteil derart mechanisch gekoppelt ist oder dafür vorgesehen und ausgebildet ist, mit dem Griffteil derart mechanisch gekoppelt zu werden, dass durch Schwenken des Griffteils relativ zu dem proximalen Ende des Spatels die Kraftübertragungseinrichtung relativ zu dem proximalen Ende des Spatels bewegbar ist, wobei die Kraftübertragungseinrichtung flexibel und aus einem elastischen Material gebildet ist.

Der Spatel ist insbesondere zur Bildung eines Laryngoskops vorgesehen und ausgebildet, das zur Intubation oder für die Mikrolarynxchirurgie oder für andere Aufgaben innerhalb der Otorhinolaryngologie verwendbar ist. Das proximale Ende des Spatels ist mit dem Griffteil auf eine dauerhafte und nicht zerstörungsfrei trennbare Weise oder auf eine zerstörungsfrei lösbare oder trennbare Weise, aber jedenfalls gelenkig mit dem Griffteil verbunden.

Der Griffteil ist zum manuellen Halten und Führen des Laryngoskops vorgesehen. Der Griffteil ist also nicht nur ein Betätigungshebel zur Steuerung einer Krümmung des Spatels, sondern ist selbst für das manuelle Halten und Führen des Laryngoskops vorgesehen und ausgebildet. Der Griffteil ist der einzige Griffteil und bildet - allenfalls zusammen mit einem proximalen Bereich des Spatels nahe dessen proximalen Ende - den einzigen Teil des Laryngoskops, der bei vorgesehener Verwendung von medizinischem Personal mit der Hand berührt, geführt und gehalten wird.

Jedes einzelne Glied des Spatels ist in sich starr, d. h. bei vorgesehener Verwendung des Spatels nicht oder nicht wesentlich elastisch oder plastisch verformbar. Jedes einzelne Gelenk kann als Festkörpergelenk oder als formschlüssiges Gelenk (beispielsweise mit einer Welle, einem Achszapfen oder Ähnlichem in einer oder mehreren korrespondierenden Lagerschalen) ausgebildet sein. Jedes Gelenk ermöglicht insbesondere ein Schwenken um eine einzige durch das Gelenk definierte Schwenkachse, wobei alle Schwenkachsen insbesondere parallel zueinander sind. Die Gelenke zwischen den Gliedern schaffen eine Verformbarkeit des gesamten Spatels. Durch Schwenken der Glieder des Spatels relativ zueinander kann die Krümmung des Spatels verändert werden.

Die insbesondere starre oder gelenkige mechanische Verbindung des distalen Endes der Kraftübertragungseinrichtung mit dem distalen Ende des Spatels und die Bewegbarkeit der Kraftübertragungseinrichtung in ihrer Längsrichtung relativ zu Gliedern des Spatels zwischen dem distalen Ende und dem proximalen Ende ermöglichen eine Variation der Krümmung des Spatels durch Bewegung der Kraftübertragungseinrichtung. Die Kopplung des proximalen Endes der Kraftübertragungseinrichtung mit dem Griffteil zum manuellen Halten des Laryngoskops ermöglicht eine Steuerung der Krümmung des Spatels durch Schwenken des Griffteils relativ zu dem proximalen Ende des Spatels. Dadurch ist insbesondere kein weiterer Hebel und keine weitere Betätigungseinrichtung erforderlich, um eine Variation der Krümmung des Spatels zu ermöglichen. Dies kann sowohl den Aufbau und damit auch die Herstellung des Spatels oder des gesamten adaptierbaren Laryngoskops vereinfachen, als auch die Handhabung durch medizinisches Personal intuitiver und damit einfacherer und sicherer machen.

Bei einem Spatel, wie er hier beschrieben ist, ist insbesondere bei einem oder mehreren oder allen der Mehrzahl von Gelenken der Abstand des Gelenks von der ersten Seite des Spatels größer als der Abstand der Kraftübertragungseinrichtung von der ersten Seite des Spatels, wobei die Kraftübertragungseinrichtung zum Übertragen einer Zugkraft ausgebildet ist.

Bei dieser Ausgestaltung ist der Abstand des Gelenks von der zweiten Seite des Spatels kleiner als der Abstand der Kraftübertragungseinrichtung von der zweiten Seite.

Bei einem Spatel, wie er hier beschrieben ist, ist insbesondere bei einem oder mehreren oder allen der Mehrzahl von Gelenken der Abstand des Gelenks von der ersten Seite des Spatels kleiner als der Abstand der Kraftübertragungseinrichtung von der ersten Seite, wobei die Kraftübertragungseinrichtung zum Übertragen einer Druck- oder Schubkraft ausgebildet ist.

Bei dieser Ausgestaltung ist der Abstand des Gelenks von der zweiten Seite des Spatels kleiner als der Abstand der Kraftübertragungseinrichtung von der zweiten Seite.

Bei einem Spatel, wie er hier beschrieben ist, sind entweder die Kraftübertragungseinrichtung nahe der ersten Seite des Spatels angeordnet und zur Übertragung einer Zugkraft vorgesehen und ausgebildet und eines oder mehrere oder alle der Mehrzahl von Gelenken nahe der zweiten Seite des Spatels angeordnet oder eines oder mehrere oder alle der Mehrzahl von Gelenken nahe der ersten Seite des Spatels angeordnet und die Kraftübertragungseinrichtung nahe der zweiten Seite des Spatels angeordnet und zur Übertragung einer Druckkraft vorgesehen und ausgebildet.

Beide Ausgestaltungen des Spatels können ein Anpassen der Krümmung des Spatels an die Anwendung und den Patienten ermöglichen. Die Anordnung der Gelenke an der bei vorgesehener Verwendung an der Zunge des Patienten anliegenden ersten Seite des Spatels kann eine atraumatische Ausgestaltung des Spatels vereinfachen. Die Kraftübertragungseinrichtung muss jedoch eine Druckkraft oder Schubkraft übertragen, um die Krümmung des Spatels zu erhöhen und entsprechend biegesteif ausgebildet und/oder geführt sein, um ein seitliches Ausbrechen zu verhindern.

Die Anordnung der Gelenke an der bei vorgesehener Verwendung dem Gaumen des Patienten zugewandten zweiten Seite kann eine einfachere Ausgestaltung der Kraftübertragungseinrichtung und ihrer Führung ermöglichen, da die Kraftübertragungseinrichtung zur Vergrößerung der Krümmung des Spatels lediglich eine Zugkraft übertragen muss.

Bei einem Spatel, wie er hier beschrieben ist, weisen insbesondere eines oder mehrere oder alle der Mehrzahl von Gliedern bezogen auf eine Schnittebene orthogonal zur Längsrichtung des Spatels einen ringförmigen Querschnitt oder einen U-förmigen Querschnitt oder einen C-förmigen Querschnitt auf.

Ein ringförmiger Querschnitt ist ein geschlossener Querschnitt, beispielsweise in Gestalt eines Kreisrings oder eines Umfangs eines Quadrats oder Rechtecks oder beliebigen anderen Polygons, optional mit abgerundeten Ecken. Ein U-förmiger oder C-förmiger Querschnitt ist ein an einer Seite (beispielsweise an der ersten Seite oder an der zweiten Seite des Spatels) offener Querschnitt, beispielsweise in Gestalt eines Teils eines Kreisrings oder eines Rands eines Rechtecks oder eines anderen Polygons, optional mit abgerundeten Ecken. Ein U-förmiger Querschnitt liegt vor, wenn die Enden des Querschnitts an dessen Öffnung parallel oder im Wesentlichen parallel zueinander angeordnet sind. Ein C-förmiger Querschnitt liegt vor, wenn die Enden des Querschnitts nahe dessen Öffnung teilweise oder vollständig einander zugewandt sind.

Ein ringförmiger oder U-förmiger oder C-förmiger Querschnitt kann eine atraumatische Ausgestaltung des Spatels ermöglichen, insbesondere wenn die Öffnung des U-förmigen oder C-förmigen Querschnitts an der bei vorgesehener Verwendung dem Gaumen des Patienten zugewandten Seite angeordnet ist. Ein ringförmiger oder U-förmiger oder C-förmiger Querschnitt kann ferner eine Führung der Kraftübertragungseinrichtung in dessen Innerem ermöglichen.

Bei einem Spatel, wie er hier beschrieben ist, ist die Kraftübertragungseinrichtung insbesondere innerhalb eines von einem oder mehreren oder allen der Mehrzahl von Gliedern zumindest teilweise umschlossenen Hohlraums angeordnet.

Die Anordnung der Kraftübertragungseinrichtung innerhalb des Hohlraums kann die Kraftübertragungseinrichtung vor einer Beschädigung schützen und eine einfache mechanische Führung der Kraftübertragungseinrichtung ermöglichen.

Ein Spatel, wie er hier beschrieben ist, umfasst insbesondere ferner eine Führungseinrichtung in einem der Mehrzahl von Gliedern, zum Führen der Kraftübertragungseinrichtung.

Bei einem Spatel, wie er hier beschrieben ist, umfasst die Führungseinrichtung insbesondere einen Teilbereich einer inneren Oberfläche des Glieds.

Die Führungseinrichtung umfasst beispielsweise einen konkaven Oberflächenabschnitt an der Innenseite des Glieds an dem die Kraftübertragungseinrichtung spiel- und reibungsarm geführt ist.

Bei einem Spatel, wie er hier beschrieben ist, umfasst die Führungseinrichtung insbesondere einen Kanal oder eine Öse.

Der Querschnitt des Kanals oder der Öse ist insbesondere so an den Querschnitt der Kraftübertragungseinrichtung angepasst, dass die Kraftübertragungseinrichtung in dem Kanal oder der Öse spiel- und reibungsarm geführt ist.

Bei einem Spatel, wie er hier beschrieben ist, ist insbesondere eines oder mehrere oder alle der Mehrzahl von Gelenken als Festkörpergelenk ausgebildet, wobei das Festkörpergelenk durch eine elastische Rückstellkraft eine Ruhekonfiguration des Gelenks definiert.

Bei einem Spatel, wie er hier beschrieben ist, ist insbesondere bei einem oder mehreren oder allen der Mehrzahl von Gelenken ein elastisches Element vorgesehen, wobei das elastische Element durch eine elastische Rückstellkraft eine Ruhekonfiguration des Gelenks definiert.

Die Ruhekonfiguration des Gelenks ist insbesondere diejenige Konfiguration oder Winkelposition des Gelenks, bei der die in dem Festkörpergelenk oder in dem elastischen Element gespeicherte elastische Energie minimal ist. Bei jedem Auslenken des Gelenks aus seiner Ruhekonfiguration oder Ruhewinkelposition nimmt das Festkörpergelenk oder das elastische Element Energie auf. Jede Auslenkung aus der Ruhekonfiguration oder Ruhewinkelposition erfolgt somit gegen die elastische Rückstellkraft des Festkörpergelenks oder des elastischen Elements. Mehrere Festkörpergelenke oder mehrere elastische Elemente des Spatels können unterschiedlich angeordnet und/oder dimensioniert sein, um unterschiedliche große Rückstellkräfte oder Rückstellmomente zu erzeugen.

Ein Spatel, wie er hier beschrieben ist, umfasst insbesondere ferner eine oder mehrere Umlenkeinrichtungen, an der die Kraftübertragungseinrichtung anliegt oder mit der die Kraftübertragungseinrichtung mechanisch verbunden ist oder die Teil der Kraftübertragungseinrichtung (80) ist, wobei die Kraftübertragungseinrichtung distal der Umlenkeinrichtung in einer ersten Richtung und proximal der Umlenkeinrichtung in einer zweiten Richtung, die sich von der ersten Richtung unterscheidet, verläuft.

Die erste Richtung, in der die Kraftübertragungseinrichtung distal einer Umlenkeinrichtung verläuft, und die zweite Richtung, in der die Kraftübertragungseinrichtung proximal derselben Umlenkeinrichtung verläuft, unterscheiden sich insbesondere um mindestens 5 Grad oder um mindestens 10 Grad oder um mindestens 20 Grad oder um mindestens 30 Grad oder um mindestens 50 Grad oder um mindestens 70 Grad. In mehreren oder allen der Mehrzahl von Gliedern können jeweils eine oder mehrere Umlenkeinrichtungen angeordnet sein. Jede Umlenkeinrichtung kann gleichzeitig eine Führungseinrichtung sein oder mit einer Führungseinrichtung integriert sein. Eine oder mehrere Umlenkeinrichtungen können insbesondere bewirken, dass Abstände zwischen der Kraftübertragungseinrichtung und den Gelenken unterschiedlich groß sind, so dass eine von der Kraftübertragungseinrichtung übertragene Kraft mit unterschiedlich großen Momenten an den Gelenken einhergeht.

Bei einem Spatel, wie er hier beschrieben ist, ist die Umlenkeinrichtung insbesondere nahe dem proximalen Ende des Spatels angeordnet.

Eine Umlenkeinrichtung nahe dem proximalen Ende des Spatels kann eine mechanische Kopplung des relativ zu dem proximalen Ende des Spatels schwenkbaren Griffteils mit der Kraftübertragungseinrichtung vereinfachen.

Bei einem Spatel, wie er hier beschrieben ist, weist die Kraftübertragungseinrichtung insbesondere eine Übersetzungseinrichtung, die eine erste Kraft und einen ersten Weg proximal der Übersetzungseinrichtung in eine zweite Kraft und einen zweiten Weg distal der Übersetzungseinrichtung übersetzt, auf.

Insbesondere sind das Verhältnis zwischen der ersten Kraft und der zweiten Kraft und das Verhältnis zwischen dem zweiten Weg und dem ersten Weg - bei Vernachlässigung von Reibung - gleich groß.

Bei einem Spatel, wie er hier beschrieben ist, umfasst die Übersetzungseinrichtung insbesondere einen Hebel, einen Flaschenzug oder ein anderes Getriebe.

Eine Übersetzungseinrichtung kann eine erwünschte Aufteilung einer auf das proximale Ende der Kraftübertragungseinrichtung ausgeübten Kraft auf die einzelnen Glieder und damit definierte Momente an den Gelenken zwischen den Gliedern oder definierte Verhältnisse zwischen den Momenten an den einzelnen Gelenken ermöglichen.

Ein Spatel für ein adaptives Laryngoskop umfasst ein proximales Ende, das mit einem Griffteil zum manuellen Halten des Laryngoskops verbunden oder verbindbar ist, ein distales Ende zum Einführen in den Rachen eines Patienten, eine erste Kette aus einer Mehrzahl von in Längsrichtung des Spatels hintereinander angeordneten und jeweils paarweise gelenkig verbundenen Gliedern, die sich von dem proximalen Ende bis zu dem distalen Ende des Spatels erstreckt, eine zweite Kette aus einer Mehrzahl von in Längsrichtung des Spatels hintereinander angeordneten und jeweils paarweise gelenkig verbundenen Gliedern, die sich von dem proximalen Ende bis zu dem distalen Ende des Spatels erstreckt, eine Mehrzahl von Abstandsbauteilen, die jeweils einen Punkt an einem Glied der ersten Kette mit einem Punkt an einem Glied der zweiten Kette gelenkig und mit einem vorbestimmten Abstand verbinden, und eine Kraftübertragungseinrichtung zum Übertragen einer Kraft, mit einem proximalen Ende und einem distalen Ende, wobei das distale Ende der Kraftübertragungseinrichtung mit dem distalen Ende des Spatels mechanisch verbunden ist, wobei die Kraftübertragungseinrichtung zwischen ihrem distalen Ende und ihrem proximalen Ende relativ zu Gliedern des Spatels in Längsrichtung der Kraftübertragungseinrichtung bewegbar ist, wobei die Kraftübertragungseinrichtung flexibel und aus einem elastischen Material gebildet ist.

Der Spatel ist insbesondere zur Bildung eines Laryngoskops vorgesehen und ausgebildet, das zur Intubation oder für die Mikrolarynxchirurgie oder für andere Aufgaben innerhalb der Otorhinolaryngologie verwendbar ist. Das proximale Ende des Spatels ist mit dem Griffteil auf eine dauerhafte und nicht zerstörungsfrei trennbare Weise oder auf eine zerstörungsfrei lösbare oder trennbare Weise, aber jedenfalls gelenkig mit dem Griffteil verbunden.

Der Griffteil ist zum manuellen Halten und Führen des Laryngoskops vorgesehen. Der Griffteil ist also nicht nur ein Betätigungshebel zur Steuerung einer Krümmung des Spatels, sondern ist selbst für das manuelle Halten und Führen des Laryngoskops vorgesehen und ausgebildet. Der Griffteil ist der einzige Griffteil und bildet - allenfalls zusammen mit einem proximalen Bereich des Spatels nahe dessen proximalen Ende - den einzigen Teil des Laryngoskops, der bei vorgesehener Verwendung von medizinischem Personal mit der Hand berührt, geführt und gehalten wird.

Die Glieder der ersten Kette und die Glieder der zweiten Kette können jeweils paarweise durch Festkörpergelenke oder durch formschlüssige Gelenke (beispielsweise mit Wellen oder Achszapfen in korrespondierenden Lagerschalen) verbunden sein. Die Abstandsbauteile sind beispielsweise durch Stege, Platten oder Rahmen, die quer zu der Längsrichtung des Spatels angeordnet sind, gebildet. Jeweils ein Ende oder Randabschnitt eines Abstandsbauteils ist mit einem oder zwei benachbarten Gliedern der ersten Kette durch ein Festkörpergelenk oder ein formschlüssiges Gelenk verbunden. Jeweils ein weiteres gegenüberliegendes Ende oder ein weiterer gegenüberliegender Randabschnitt des Abstandsbauteils ist mit einem oder zwei benachbarten Gliedern der zweiten Kette durch ein Festkörpergelenk oder ein formschlüssiges Gelenk verbunden.

Das distale Ende der Kraftübertragungseinrichtung ist mit dem distalen Ende des Spatels insbesondere mechanisch starr oder gelenkig verbunden. Das proximale Ende der Kraftübertragungseinrichtung kann mit dem Griffteil zum Halten des Laryngoskops dauerhaft und nicht zerstörungsfrei lösbar oder trennbar oder aber in einer zerstörungsfrei lösbaren oder trennbaren Weise verbunden sein. Das proximale Ende der Kraftübertragungseinrichtung ist mit dem Griffteil insbesondere so mechanisch gekoppelt, dass ein Schwenken des Griffteils relativ zu dem proximalen Ende des Spatels ein Bewegen der Kraftübertragungseinrichtung relativ zu Gliedern des Spatels bewirkt. Mittels der Kraftübertragungseinrichtung können unmittelbar oder mittelbar Kräfte oder Momente auf die Glieder der Ketten ausgeübt werden. Dadurch kann mittels der Kraftübertragungseinrichtung eine Krümmung des Spatels beeinflusst werden.

Ein Spatel, wie er hier beschrieben ist, umfasst insbesondere ferner eine Führungseinrichtung an einem der Mehrzahl von Abstandsbauteilen, zum Führen der Kraftübertragungseinrichtung.

Bei einem Spatel, wie er hier beschrieben ist, ist insbesondere an einem ersten Abstandsbauteil der Mehrzahl von Abstandsbauteilen eine erste Führungseinrichtung zum Führen der Kraftübertragungseinrichtung vorgesehen, wobei an einem zweiten Abstandsbauteil der Mehrzahl von Abstandsbauteilen eine zweite Führungseinrichtung zum Führen der Kraftübertragungseinrichtung vorgesehen ist, wobei ein erstes Verhältnis zwischen dem Abstand der ersten Führungseinrichtung von einem ersten Ende des ersten Abstandsbauteils, das dem zugeordneten Glied der ersten Kette zugewandt ist, und dem Abstand der ersten Führungseinrichtung von einem zweiten Ende des ersten Abstandsbauteils, das dem zugeordneten Glied der zweiten Kette zugewandt ist, verschieden ist von einem zweiten Verhältnis zwischen dem Abstand der zweiten Führungseinrichtung von einem ersten Ende des zweiten Abstandsbauteils, das dem zugeordneten Glied der ersten Kette zugewandt ist, und dem Abstand der zweiten Führungseinrichtung von einem zweiten Ende des zweiten Abstandsbauteils, das dem zugeordneten Glied der zweiten Kette zugewandt ist.

Die unterschiedliche Anordnung der Führungseinrichtungen an den Abstandsbauteilen kann bewirken, dass eine auf das proximale Ende der Kraftübertragungseinrichtung ausgeübte Kraft unterschiedliche Kräfte oder Momente an den Abstandsbauteilen bewirkt.

Ein Laryngoskop umfasst einen Spatel, wie er hier beschrieben ist, und einen Griffteil, der mit dem proximalen Ende des Spatels verbunden oder verbindbar ist.

Bei einem Laryngoskop, wie es hier beschrieben ist, ist das proximalen Ende des Spatels insbesondere gelenkig mit dem Griffteil verbunden oder verbindbar, wobei das proximale Ende der Kraftübertragungseinrichtung derart mit dem Griffteil gekoppelt oder koppelbar ist, dass durch Schwenken des Griffteils relativ zu dem proximalen Ende des Spatels eine Zugkraft oder eine Druck- oder Schubkraft auf die Kraftübertragungseinrichtung ausgeübt werden kann.

### Kurzbeschreibung der Figuren

Nachfolgend werden Ausführungsformen anhand der beigefügten Figuren näher erläutert. Es zeigen:
- Figur 1: eine schematische Darstellung eines Laryngoskops;
- Figur 2: eine schematische Schnittdarstellung des Laryngoskops aus Figur 1;
- Figur 3: eine weitere schematische Schnittdarstellung des Laryngoskops aus den Figuren 1 und 2;
- Figur 4: eine weitere schematische Schnittdarstellung des Laryngoskops aus den Figuren 1 bis 3;
- Figur 5: eine schematische Schnittdarstellung eines weiteren Laryngoskops;
- Figur 6: eine weitere schematische Schnittdarstellung des Laryngoskops aus Figur 5;
- Figur 7: eine schematische Schnittdarstellung eines weiteren Laryngoskops;
- Figur 8: eine schematische Schnittdarstellung eines weiteren Laryngoskops;

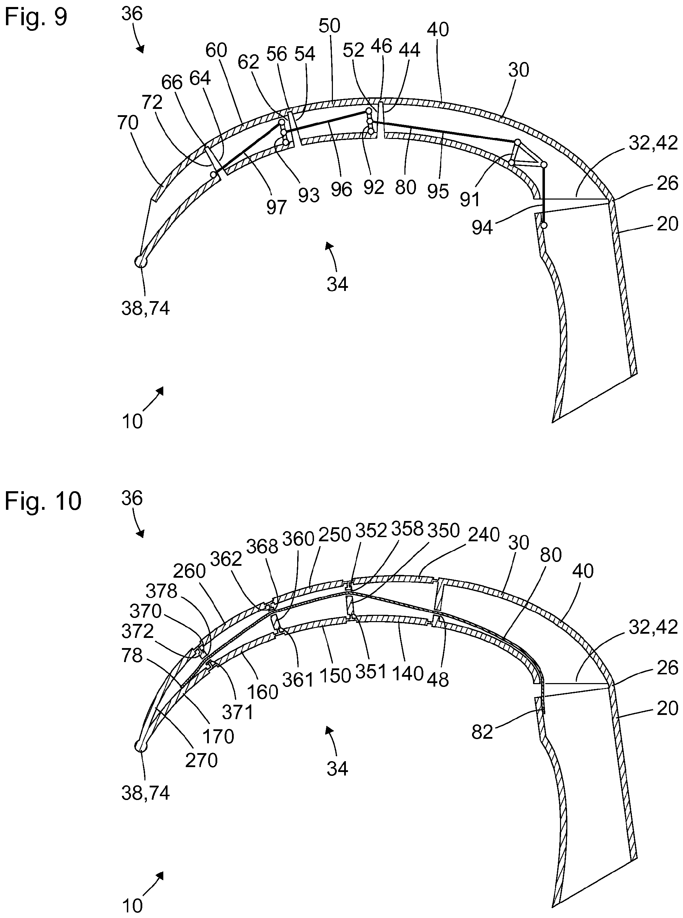

- Figur 9: eine schematische Schnittdarstellung eines weiteren Laryngoskops;
- Figur 10: eine schematische Schnittdarstellung eines weiteren Laryngoskops

### Beschreibung der Ausführungsformen

Figur 1 zeigt eine schematische Darstellung eines Laryngoskops 10 mit einem Griffteil 20 und einem Spatel 30. Der Griffteil 20 ist durch ein Gelenk 26 mit einem proximalen Ende 32 des Spatels 40 verbunden. Das Gelenk 26 ermöglicht ein Schwenken des Griffteils 20 relativ zu dem Spatel 30, insbesondere zu dem proximalen Ende 32 des Spatels 30 um eine Schwenkachse orthogonal zu der Zeichenebene der Figur 1. Das Gelenk 26 kann - wie in Figur 1 angedeutet - als Festkörpergelenk (oft auch als Foliengelenk bezeichnet) ausgebildet sein. Alternativ kann das Gelenk als formschlüssiges Gelenk ausgebildet sein, beispielsweise mit einer Welle oder Achszapfen und einer oder mehreren korrespondierenden Lagerschalen.

Der Griffteil 20 und der Spatel 30 können dauerhaft und nicht zerstörungsfrei lösbar miteinander verbunden sein, wie im Fall des in Figur 1 angedeuteten monolithischen oder weitgehend monolithischen Aufbaus des Laryngoskops 10 mit dem Festkörpergelenk 26. Alternativ und abweichend davon kann eine Kupplung zur zerstörungsfrei lösbaren oder trennbaren mechanischen Verbindung zwischen dem Griffteil und dem Spatel 30 vorgesehen sein. Diese Kupplung kann mit dem Gelenk 26 integriert oder an dem dem Spatel zugewandten Ende des Griffteils 20 oder an dem dem Griffteil 20 zugewandten Ende 32 des Spatels 30 vorgesehen sein.

Der Spatel 30 weist eine erste, bei vorgesehener Verwendung des Laryngoskops 10 an der Zunge und/oder dem Zungengrund eines Patienten anliegende Seite 34 und eine zweite, bei vorgesehener Verwendung des Laryngoskops 10 dem Gaumen des Patienten zugewandte Seite 36 auf. Der Spatel 30 erstreckt sich bis zu einem distalen Ende 38, das insbesondere für eine atraumatische Verwendung mit möglichst großen Krümmungsradien ausgebildet ist.

Der Spatel 30 umfasst mehrere Glieder 40, 50, 60, 70, die durch Gelenke 46, 56, 66 miteinander verbunden sind. Ein proximales Ende 42 eines ersten Glieds 40 bildet das proximale Ende 32 des Spatels 30. Ein distales Ende 44 des ersten Glieds 40 ist durch ein erstes Gelenk 46 mit einem proximalen Ende 52 eines zweiten Glieds 50 verbunden. Ein distales Ende 54 des zweiten Glieds 50 ist durch ein zweites Gelenk 56 mit einem proximalen Ende 62 eines dritten Glieds 60 verbunden. Ein distales Ende 64 des dritten Glieds 60 ist durch ein drittes Gelenk 66 mit einem proximalen Ende 72 eines vierten Glieds 70 verbunden. Ein distales Ende 74 des vierten Glieds 70 bildet das distale Ende 38 des Spatels 30.

Jedes einzelne Glied 40, 50, 60, 70 des Spatels 30 erstreckt sich von der bei vorgesehener Verwendung des Laryngoskops 10 an der Zunge des Patienten anliegenden ersten Seite 34 bis zu der bei vorgesehener Verwendung dem Gaumen des Patienten zugewandten zweiten Seite 36 des Spatels 30. Jedes Glied 40, 50, 60, 70 ist in sich starr ausgebildet, wird also durch die bei vorgesehener Verwendung des Laryngoskops 10 austretenden Kräfte und Momente nicht oder nicht wesentlich verformt.

Die Gelenke 46, 56, 66 ermöglichen Schwenkbewegungen benachbarter Glieder 40, 50, 60, 70 relativ zueinander um zugeordnete Schwenkachsen orthogonal zu der Zeichenebene der Figur 1. Bei dem dargestellten Beispiel sind die Gelenke 46, 56, 66 als Festkörpergelenke ausgebildet. Alternativ können die Gelenke 46, 56, 66 beispielsweise als formschlüssige Gelenke mit Wellen und/oder Achszapfen in korrespondierenden Lagerschalen ausgebildet sein.

Die Gelenke 46, 56, 66 sind an der zweiten Seite 36 angeordnet. Zwischen den Gliedern 40, 50, 60, 70 erstrecken sich von der ersten Seite 34 bis zu den Gelenken 46, 56, 66 Spalte, die eine Vergrößerung der Krümmung des Spatels 30 ermöglichen. Alternativ und abweichend von der Darstellung in Figur 1 können die Glieder 40, 50, 60, 70 überlappend ausgebildet sein, so keine Spalte zwischen den Gliedern 40, 50, 60, 70 vorliegen.

In den Spalten zwischen den Gliedern 40, 50, 60, 70 ist eine Kraftübertragungseinrichtung 80 sichtbar, deren proximales Ende mit dem Griffteil 20 und deren distales Ende mit dem vierten Glied 70 und damit mit dem distalen Ende 38 des Spatels 30 mechanisch starr verbunden ist. Die Kraftübertragungseinrichtung 80 ist aufgrund ihres Materials und/oder ihres Querschnitts flexibel, und aus einem elastischen Material gebildet. In Längsrichtung weist die Kraftübertragungseinrichtung eine geringe Elastizität auf, so dass sie bei den bei vorgesehener Verwendung des Laryngoskops 10 auftretenden Kräften nicht wesentlich gedehnt oder gestaucht wird.

Figur 2 zeigt eine schematische Darstellung eines Schnitts durch das Laryngoskop 10 entlang einer Ebene parallel zu der Zeichenebene der Figur 1. Der Griffteil 20 oder der Spatel 30 oder das gesamte Laryngoskop 10 kann spiegelsymmetrisch zu der Schnittebene der Figur 2 ausgebildet sein. Die in Figur 2 gezeigte Konfiguration des Laryngoskops 10 entspricht der in Figur 1 gezeigten Konfiguration.

In Figur 2 ist erkennbar, dass die Glieder 40, 50, 60, 70 des Spatels 30 jeweils einen Hohlraum, der an beiden Enden offen ist, umschließen. Der gesamte Spatel weist somit einen Hohlraum auf, der - von den Spalten an den Gelenken 46, 56, 66 zwischen den Gliedern 40, 50, 60, 70 abgesehen - von den Gliedern 40, 50, 60, 70 weitgehend umschlossen ist. Die Kraftübertragungseinrichtung 80 ist in diesem Hohlraum angeordnet.Ein Wandabschnitt jedes Glieds 40, 50, 60, 70 an der bei vorgesehener Verwendung an der Zunge des Patienten anliegenden ersten Seite 34 bildet eine Führungseinrichtung 48, 58, 68, 78, an der die Kraftübertragungseinrichtung 80 anliegt.

Das proximale Ende 82 der Kraftübertragungseinrichtung 80 ist mit dem Griffteil 20 an einer von dem Gelenk 26 zwischen dem Griffteil 20 und dem Spatel 30 beabstandeten Stelle verbunden. In dem Spalt zwischen dem Griffteil 20 und dem proximalen Ende 32 des Spatels 30 ist die Kraftübertragungseinrichtung 80 von dem Gelenk 26 beabstandet. Das distale Ende 87 der Kraftübertragungseinrichtung 80 ist mit dem vierten Glied 40 an einer von dem Gelenk 66 zwischen dem dritten Glied 60 und dem vierten Glied 70 beabstandeten Stelle mechanisch verbunden. In dem Spalt zwischen dem dritten Glied 60 und dem vierten Glied 70 ist die Kraftübertragungseinrichtung 80 von dem Gelenk 26 beabstandet. Bei vorgesehener Verwendung des Laryngoskops 10 überträgt die Kraftübertragungseinrichtung 80 eine Zugkraft von dem Griffteil 20 zu dem distalen Ende 38 des Spatels 30, nämlich zu dessen vierten Glied 70. Dadurch, dass die Kraftübertragungseinrichtung 80 bei vorgesehener Verwendung des Laryngoskops 10 unter Zugspannung steht, liegt sie wie in Figur 2 angedeutet an den Führungseinrichtungen 48, 58, 68, 78 an. Um vor allem in den Bereichen zwischen dem Griffteil 20 und dem Spatel 30 und zwischen den Gliedern 40, 50, 60, 70 des Spatels 30 eine elastische Verformung der Kraftübertragungseinrichtung zu ermöglichen, ist die Kraftübertragungseinrichtung insbesondere aus einem elastischen Material gebildet und/oder weist einen niedrigen Querschnitt, also beispielsweise die Gestalt eines Bandes auf.

Figur 3 zeigt eine weitere schematische Darstellung eines Schnitts durch das anhand der Figuren 1 und 2 dargestellte Laryngoskop 10. Die Schnittebene der Figur 3 entspricht der Schnittebene der Figur 2. Das Laryngoskop 10 ist in Figur 3 in einer Konfiguration dargestellt, die sich von der in den Figuren 1 und 2 dargestellten Konfiguration unterscheidet.

Bei der in Figur 3 gezeigten Konfiguration ist der Griffteil 20 des Laryngoskops 10 zu dem proximalen Ende 32 des Spatels 30 hin geschwenkt, und der Spatel 30 ist gegenüber der in Figur 2 dargestellten Konfiguration gestreckt, weist also eine geringere Gesamtkrümmung auf. Dazu sind die Glieder 40, 50, 60, 70 in den Gelenken 46, 56, 66 jeweils paarweise voneinander weggeschwenkt, so dass die Spalte zwischen den Gliedern 40, 50, 60, 70 vergrößert sind.

Figur 4 zeigt eine weitere schematische Darstellung eines Schnitts durch das anhand der Figuren 1 bis 3 dargestellte Laryngoskop 10. Die Schnittebene der Figur 4 entspricht den Schnittebenen der Figuren 2 und 3. In Figur 4 ist eine Konfiguration dargestellt, die sich von den in den Figuren 1 bis 3 dargestellten Konfigurationen unterscheidet.

Bei der in Figur 4 gezeigten Konfiguration des Laryngoskops 10 ist der Griffteil 20 gegenüber den in den Figuren 1 bis 3 gezeigten Konfigurationen von dem proximalen Ende 32 des Spatels 30 weggeschwenkt. Aufgrund der von dem Gelenk 26 zwischen dem Griffteil 20 und dem proximalen Ende 32 des Spatels 30 beabstandeten Befestigung des proximalen Endes 82 der Kraftübertragungseinrichtung erzeugt diese Schwenkbewegung hin zu der in Figur 4 gezeigten Winkelposition des Griffteils 20 eine Zugkraft in der Kraftübertragungseinrichtung 80 und eine Verlagerung der Kraftübertragungseinrichtung 80 zu dem proximalen Ende 32 des Spatels 30 hin. Dies wiederum bewirkt ein Schwenken des zweiten Glieds 50 zu dem ersten Glied 40 hin, ein Schwenken des dritten Glieds 60 zu dem zweiten Glied 50 hin und ein Schwenken des vierten Glieds 70 zu dem dritten Glied 60 hin. Es entsteht die in Figur 4 gezeigte Konfiguration mit einer gegenüber den anhand der Figuren 1 bis 3 dargestellten Konfigurationen vergrößerten Krümmung des Spatels 30.

Ausgehend von der in Figur 4 gezeigten Konfiguration kann eine von einer Zunge oder einem Zungengrund eines Patienten auf die erste Seite 34 des Spatels 30 ausgeübte Kraft eine Streckung des Spatels 30 zu der in den Figuren 1 und 2 gezeigten Konfiguration und weiter bis zu der in Figur 3 gezeigten Konfiguration bewirken, wenn auf den Griffteil 20 keine entsprechende Gegenkraft ausgeübt wird. Im Ergebnis stellt sich bei dem anhand der Figuren 1 bis 4 dargestellten Laryngoskop 10 jederzeit eine Konfiguration ein, bei der ein Gleichgewicht der Kräfte und Momente auf den Griffteil 20 (manuell erzeugt durch medizinisches Personal) und auf die Glieder 40, 50, 60, 70 des Spatels 30 (erzeugt durch Zunge und/oder Zungengrund eines Patienten) vorliegen.

Figur 5 zeigt eine schematische Darstellung eines Schnitts durch ein weiteres Laryngoskop 10, das in einigen Merkmalen, Eigenschaften und Funktionen dem anhand der Figuren 1 bis 4 dargestellten Laryngoskop ähnelt. Die Schnittebene der Figur 5 entspricht den Schnittebenen der Figuren 2 bis 4. Nachfolgend sind insbesondere Merkmale, Eigenschaften und Funktionen des in Figur 5 gezeigten Laryngoskops beschrieben, in denen dieses sich von dem anhand der Figuren 1 bis 4 dargestellten Laryngoskop unterscheidet.

Auch das in Figur 5 gezeigte Laryngoskop 10 weist einen Griffteil 20 und einen Spatel 30 aus mehreren Gliedern 40, 50, 60, 70 auf, die durch Gelenke 26, 46, 56, 66 miteinander verbunden sind. Ähnlich wie bei dem anhand der Figuren 1 bis 4 dargestellten Laryngoskop sind auch bei dem in Figur 5 gezeigten Laryngoskop die Gelenke 26, 46, 56, 66 als Festkörpergelenke ausgebildet, können jedoch alternativ und abweichend von der Darstellung in Figur 5 beispielsweise als formschlüssige Gelenke mit Wellen oder Achszapfen, die in korrespondierenden Lagerschalen gelagert sind, ausgebildet sein.

Anders als bei dem anhand der Figuren 1 bis 4 dargestellten Laryngoskop sind bei dem in Figur 5 gezeigten Laryngoskop die Gelenke 26, 46, 56, 66 nicht an der zweiten Seite 36, sondern an der bei vorgesehener Verwendung des Laryngoskops 10 an der Zunge oder dem Zungengrund des Patienten anliegenden ersten Seite 34 angeordnet.

Wie bei dem anhand der Figuren 1 bis 4 dargestellten Laryngoskop ist auch bei dem in Figur 5 gezeigten Laryngoskop 10 eine Kraftübertragungseinrichtung 80 innerhalb des von den Gliedern 40, 50, 60, 70 weitgehend umschlossenen Hohlraums angeordnet. Das in Figur 5 gezeigte Laryngoskop 10 unterscheidet sich von dem anhand der Figuren 1 bis 4 dargestellten Laryngoskop jedoch ferner dadurch, dass die Kraftübertragungseinrichtung 80 nicht an der ersten Seite 34, sondern an der bei vorgesehener Verwendung des Laryngoskops 10 dem Gaumen zugewandten zweiten Seite 36 des Spatels 30 angeordnet ist. Entsprechend sind ein proximales Ende 82 der Kraftübertragungseinrichtung 80 an einer von dem Gelenk 26 zwischen Griffteil 20 und proximalem Ende 32 des Spatels 30 beabstandeten Stelle mit dem Griffteil 20 und ein distales Ende 87 der Kraftübertragungseinrichtung 80 an einer von dem Gelenk 66 zwischen dem dritten Glied 60 und dem vierten Glied 70 beabstandeten Stelle an der zweiten Seite 36 des Spatels 30 mit dem vierten Glied 70 mechanisch verbunden.

Das in Figur 5 gezeigte Laryngoskop 10 weist Führungseinrichtungen 48, 58, 68 für die Kraftübertragungseinrichtung 80 auf, die anders als bei dem anhand der Figuren 1 bis 4 dargestellten Laryngoskop einen lediglich zwischen den Gliedern 40, 50, 60, 70 unterbrochenen Kanal bilden, in dem die Kraftübertragungseinrichtung 80 spiel- und reibungsarm geführt ist. Auch das anhand der Figuren 1 bis 4 dargestellte Laryngoskop kann abweichend von den Darstellungen in den Figuren 2 bis 4 kanalförmige Führungseinrichtungen für die Kraftübertragungseinrichtung aufweisen.

Bei dem in Figur 5 gezeigten Laryngoskop 10 bewirkt ein Schwenken des Griffteils 20 ausgehend von der in Figur 5 gezeigten Konfiguration zu dem proximalen Ende 32 des Spatels 30 hin eine Druck- oder Schubkraft in der Kraftübertragungseinrichtung 80 und eine Bewegung der Kraftübertragungseinrichtung 80 relativ zu dem ersten Glied 40 des Spatels 30 nach distal. Die Führung der Kraftübertragungseinrichtung 80 durch die kanalförmigen Führungseinrichtungen 48, 58, 68 und eine ausreichend biegesteife Ausführung der Kraftübertragungseinrichtung 80 verhindern ein seitliches Ausweichen der Kraftübertragungseinrichtung 80 bei den bei vorgesehener Verwendung auftretenden Kräften.

Figur 6 zeigt eine schematische Darstellung eines weiteren Schnitts durch das anhand der Figur 5 dargestellte Laryngoskop. Die Schnittebene der Figur 6 entspricht der Schnittebene der Figur 5. Die in Figur 6 gezeigte Konfiguration unterscheidet sich von der in Figur 5 gezeigten Konfiguration des Laryngoskops 10.

Bei der in Figur 6 gezeigten Konfiguration ist der Griffteil 20 zu dem proximalen Ende 32 des Spatels 30 hin geschwenkt. Dies bewirkt eine Druck- oder Schubkraft in der Kraftübertragungseinrichtung 80 und eine Bewegung der Kraftübertragungseinrichtung 80 relativ zu dem ersten Glied 40, dem zweiten Glied 50 und dem dritten Glied 60 des Spatels 30 nach distal und eine Vergrößerung der Krümmung des Spatels 30. Folglich sind bei der in Figur 6 gezeigten Konfiguration die Spalte zwischen den Gliedern 40, 50, 60, 70, die sich von der bei vorgesehener Verwendung dem Gaumen des Patienten zugewandten zweiten Seite 36 zu den Gelenken 46, 56, 66 erstrecken, verbreitert.

Wie bei dem anhand der Figuren 1 bis 4 dargestellten Laryngoskop kann auch bei dem in den Figuren 5 und 6 gezeigten Laryngoskop 10 die Krümmung des Spatels 30 durch Schwenken des Griffteils 20 relativ zu dem proximalen Ende 32 des Spatels 30 eingestellt werden. Es stellt sich eine Form des Spatels 30 ein, bei der ein Gleichgewicht zwischen den manuell an dem Griffteil 20 erzeugten Kräften und Momenten, den auf die Glieder 40, 50, 60, 70 des Spatels 30 wirkenden Kräften und Momenten und gegebenenfalls Rückstellkräften der Gelenke 26, 46, 56, 66 vorliegt.

Figur 7 zeigt eine schematische Darstellung eines Schnitts durch ein weiteres Laryngoskop 10, das in einigen Merkmalen, Eigenschaften und Funktionen den anhand der Figuren 1 bis 6 dargestellten Laryngoskopen ähnelt. Die Schnittebene der Figur 7 entspricht den Schnittebenen der Figuren 2 bis 6. Nachfolgend sind insbesondere Merkmale, Eigenschaften und Funktionen des in Figur 7 gezeigten Laryngoskops beschrieben, in denen dieses sich von den anhand der Figuren 1 bis 6 dargestellten Laryngoskopen unterscheidet.

Das in Figur 7 gezeigte Laryngoskop 10 unterscheidet sich von dem anhand der Figuren 1 bis 4 dargestellten Laryngoskop insbesondere dadurch, dass eine Umlenkeinrichtung 84 für die Kraftübertragungseinrichtung 80 vorgesehen ist. Die Umlenkeinrichtung 84 ist bei dem dargestellten Beispiel in dem ersten Glied 40 des Spatels 30 angeordnet. Die Umlenkeinrichtung 84 weist näherungsweise die Gestalt eines Kreissegments auf, an dessen Umfang die Kraftübertragungseinrichtung 80 anliegt, und dessen Spitze durch ein Festkörpergelenk 85 mit einer inneren Oberfläche des ersten Glieds 40 verbunden ist. Bei dem dargestellten Beispiel ist auch die Kraftübertragungseinrichtung 80 näherungsweise punktförmig stoffschlüssig mit der Umlenkeinrichtung 84 verbunden.

Die Umlenkeinrichtung 84 bewirkt eine Umlenkung der Richtung, in der die Kraftübertragungseinrichtung 80 verläuft. Die Umlenkeinrichtung 84 kann die Reibung zwischen der Kraftübertragungseinrichtung 80 und dem ersten Glied 40 vermindern. Ferner kann das Festkörpergelenk 85 zwischen der Umlenkeinrichtung 84 und dem ersten Glied 40 abweichend von der Darstellung in Figur 7 von dem Krümmungsmittelpunkt der Oberfläche der Umlenkeinrichtung 84, an der die Kraftübertragungseinrichtung 80 anliegt, beabstandet sein. Dies kann eine Übersetzung bewirken, bei der eine erste Kraft und ein erster Weg an der Kraftübertragungseinrichtung 80 proximal der Umlenkeinrichtung 84 in eine zweite Kraft und einen zweiten Weg distal der Umlenkeinrichtung 84 übersetzt werden. Diese Übersetzung kann von der Position der Umlenkeinrichtung 84 und damit von der Position der Kraftübertragungseinrichtung 80 abhängig sein.

Abweichend von der Darstellung in Figur 7 kann die Kraftübertragungseinrichtung 80 nur reibschlüssig oder gar nicht mit der Umlenkeinrichtung 84 verbunden sein. Abweichend von der Darstellung in Figur 7 kann ferner die Umlenkeinrichtung 84 als Rolle oder Rad oder Rollensegment oder Radsegment oder in Gestalt einer Kurvenscheibe ausgebildet sein. Ferner kann die Umlenkeinrichtung 84 abweichend von der Darstellung in Figur 7 über eine Welle oder einen oder mehrere Achszapfen und korrespondierende Lagerschalen mit dem ersten Glied 40 verbunden sein. Abweichend von der Darstellung in Figur 7 kann die Umlenkeinrichtung 84 ferner in dem zweiten Glied 50 oder in dem dritten Glied 60 angeordnet sein. Ferner können abweichend von der Darstellung in Figur 7 mehrere Umlenkeinrichtungen 84 vorgesehen sein.

Figur 8 zeigt eine schematische Darstellung eines Schnitts durch ein weiteres Laryngoskop 10, das in einigen Merkmalen, Eigenschaften und Funktionen den anhand der Figuren 1 bis 7 dargestellten Laryngoskopen ähnelt. Die Schnittebene der Figur 8 entspricht den Schnittebenen der Figuren 2 bis 7. Nachfolgend sind insbesondere Merkmale, Eigenschaften und Funktionen des in Figur 8 gezeigten Laryngoskops beschrieben, in denen dieses sich von den anhand der Figuren 1 bis 7 dargestellten Laryngoskopen unterscheidet.

Das in Figur 8 gezeigte Laryngoskop 10 unterscheidet sich insbesondere von dem anhand der Figuren 1 bis 4 dargestellten Laryngoskop insbesondere dadurch, dass an den einander zugewandten Enden 44, 52, 54, 62, 64, 72 der Glieder 40, 50, 60, 70 Führungseinrichtungen 48, 58, 68, 78 in Gestalt von Ösen mit unterschiedlichen Abständen von den Gelenken 46, 56, 66 vorgesehen sind. Bei dem dargestellten Beispiel sind an einander gegenüberliegenden Enden 44, 52 oder 54, 62 oder 64, 72 zweier benachbarter Glieder 40, 50, 60, 70 immer zwei einander gegenüberliegende Ösen 48, 58, 68, 78 vorgesehen. Abweichend davon kann an jedem Übergang zwischen zwei benachbarten Gliedern 40, 50, 60, 70 nur eine Führungseinrichtung 48, 58, 68, 78 an einem Ende 44, 52, 54, 62, 64, 72 von einem der beiden benachbarten Glieder 40, 50, 60, 70 vorgesehen sein.

Die unterschiedlichen Abstände der Führungseinrichtungen 48, 58, 68, 78 von den Gelenken 46, 56, 66 zwischen den Gliedern 40, 50, 60, 70 bewirken, dass eine Zugkraft in der Kraftübertragungseinrichtung 80 unterschiedliche den Spatel 30 krümmende Momente an den Gelenken 46, 56, 66 erzeugt. Beispielsweise bewirkt die in Figur 8 angedeutete Anordnung der Führungseinrichtungen 48, 58, 68, 78, dass ein Schwenken des Griffteils 20 relativ zu dem proximalen Ende 32 des Spatels 30 die Krümmung an dem dritten Gelenk 66 etwas stärker als an dem zweiten Gelenk 56 und deutlich stärker als an dem ersten Gelenk 46 beeinflusst. Damit kann beispielsweise kompensiert werden, dass bei vorgesehener Verwendung des Laryngoskops 10 der Zungengrund (nahe dem distalen Ende 38 des Spatels 30) dem Spatel 30 eine größere Kraft entgegensetzt als andere Teile der Zunge oder dass vor allem mit dem distalen Ende 38 des Spatels des Laryngoskops 10 eine Kraft ausgeübt werden soll.

Figur 9 zeigt eine schematische Darstellung eines Schnitts durch ein weiteres Laryngoskop 10, das in einigen Merkmalen, Eigenschaften und Funktionen den anhand der Figuren 1 bis 8 dargestellten Laryngoskopen ähnelt. Die Schnittebene der Figur 9 entspricht den Schnittebenen der Figuren 2 bis 8. Nachfolgend sind insbesondere Merkmale, Eigenschaften und Funktionen des in Figur 9 gezeigten Laryngoskops 10 beschrieben, in denen dieses sich von den anhand der Figuren 1 bis 8 dargestellten Laryngoskopen unterscheidet.

Das in Figur 9 gezeigte Laryngoskop 10 unterscheidet sich insbesondere von den anhand der Figuren 1 bis 4, 7 und 8 gezeigten Laryngoskopen insbesondere dadurch, dass die Kraftübertragungseinrichtung 80 mehrere Hebel 91, 92, 93 umfasst, die mit dem Griffteil 20, untereinander und mit dem vierten Glied 70 durch Zugstangen 94, 95, 96, 97 oder andere wenig dehnungselastische Zugelemente miteinander verbunden sind. Eine erste Zugstange 94 koppelt den Griffteil 20 mit einem ersten Hebel 91 in dem ersten Glied 40. Eine zweite Zugstange 95 koppelt den ersten Hebel 91 in dem ersten Glied 40 mit einem zweiten Hebel 92 in dem zweiten Glied 50. Eine dritte Zugstange 96 koppelt den zweiten Hebel 92 in dem zweiten Glied 50 mit einem dritten Hebel 93 in dem dritten Glied 60. Eine vierte Zugstange 97 koppelt den dritten Hebel 93 in dem dritten Glied 60 mit dem vierten Glied 70.

Ein von dem ersten Hebel 91 beabstandetes proximales Ende der ersten Zugstange 94 ist an einem von dem Gelenk 26 zwischen dem Griffteil 20 und dem proximalen Ende 32 des Spatels 30 beabstandeten Ort mit dem Griffteil 20 mechanisch verbunden. Das von dem dritten Hebel 93 abgewandte distale Ende der Zugstange 97 ist an einem von dem Gelenk zwischen dem dritten Glied 60 und dem vierten Glied 70 beabstandeten Ort nahe der ersten Seite 34 mit dem vierten Glied 70 verbunden. Bei dem dargestellten Beispiel sind der erste Hebel 91 als geknickter Hebel oder Winkelhebel und der zweite Hebel 92 und der dritte Hebel 93 als gerade Hebel ausgebildet. Der erste Hebel 91, der zweite Hebel 92 und der dritte Hebel 93 sind jeweils nahe der bei vorgesehener Verwendung des an der Zunge des Patienten anliegenden ersten Seite 34 des Spatels 30 und damit beabstandet von den Gelenken 46, 56, 66 an der zweiten Seite 36 des Spatels 30 angelenkt.

Der als Winkelhebel ausgebildete erste Hebel 91 bewirkt eine Umlenkung der von der Kraftübertragungseinrichtung 80 übertragenen Kraft. Alle Hebel 91, 92, 93 bewirken durch unterschiedliche Abstände der Koppelstellen der Zugstangen 94, 95, 96, 97 von den Schwenkachsen der Hebel 91, 92, 93 jeweils eine Übersetzung einer ersten Kraft und eines ersten Wegs proximal des Hebels 91, 92, 93 in eine zweite Kraft und einen zweiten Weg distal des Hebels 91, 92, 93. Im reibungsfreien Fall entspricht das Verhältnis zwischen der ersten Kraft und der zweiten Kraft dem Verhältnis zwischen dem zweiten Weg und dem ersten Weg. Schwenkbewegungen der Glieder 40, 50, 60, 70 relativ zueinander und der Hebel 91, 92, 93 relativ zu den Gliedern 40, 50, 60 und damit variierende Zugrichtungen der Zugstangen 94, 95, 96, 97 können die von den Hebeln 91, 92, 93 bewirkten Übersetzungsverhältnisse verändern.

Übersetzungen mittels Hebeln 91, 92, 93 oder - abweichend von der Darstellung in Figur 9 - durch Flaschenzüge oder andere Getriebe innerhalb der Kraftübertragungseinrichtung 80 können ähnlich wie bei dem anhand der Figur 8 dargestellten Laryngoskop einen entlang des Spatels 30 variierenden Einfluss einer Schwenkbewegung des Griffteils 20 relativ zu dem proximalen Ende 32 des Spatels 30 auf die Krümmung des Spatels 30 bewirken. Anders ausgedrückt können Hebel 91, 92, 93, 94 oder andere Getriebe bewirken, dass eine Schwenkbewegung des Griffteils 20 relativ zu dem proximalen Ende 32 des Spatels die Krümmung des Spatels 30 an einem ersten Gelenk 46, 56, 66 stärker oder schwächer beeinflusst als an einem zweiten Gelenk 46, 56, 66.

Figur 10 zeigt eine schematische Darstellung eines Schnitts durch ein weiteres Laryngoskop 10, das in einigen Merkmalen, Eigenschaften und Funktionen den anhand der Figu-ren 1 bis 9 dargestellten Laryngoskopen ähnelt. Die Schnittebene der Figur 10 entspricht den Schnittebenen der Figuren 2 bis 9. Nachfolgend sind insbesondere Merkmale, Eigenschaften und Funktionen des in Figur 10 gezeigten Laryngoskops 10 beschrieben, in denen dieses sich von den anhand der Figuren 1 bis 9 dargestellten Laryngoskopen unterscheidet.

Das in Figur 10 gezeigte Laryngoskop 10 weist ähnlich wie die anhand der Figuren 1 bis 9 dargestellten Laryngoskope einen Griffteil 20 und einen Spatel 30, dessen proximales Ende 32 durch ein Gelenk 26 mit dem Griffteil 20 mechanisch gelenkig verbunden ist, auf. An einem proximalen Abschnitt 40 des Spatels 30 schließt sich eine erste Kette von Gliedern 140, 150, 160, 170 an der bei vorgesehener Verwendung des Laryngoskops 10 an der Zunge eines Patienten anliegenden ersten Seite 34 an. Ferner schließt sich an den proximalen Abschnitt 40 des Spatels 30 eine zweite Kette von Gliedern 240, 250, 260, 270 an der bei vorgesehener Verwendung des Laryngoskops dem Gaumen des Patienten zugewandten Seite 36 an. Die zweite Kette von Gliedern 240, 250, 260, 270 ist im Wesentlichen parallel zu der ersten Kette von Gliedern 140, 150, 160, 170, wobei der Abstand beider Ketten nach distal abnimmt. Die äußerst distalen Glieder 170, 270 sind an ihren distalen Enden miteinander mechanisch starr verbunden und bilden das distale Ende 38 des Spatels 30. Zwischen dem proximalen Abschnitt 40 des Spatels 30 und den Gliedern 140, 150, 160, 170, 240, 250, 260, 270 sind Gelenke vorgesehen, die bei dem dargestellten Beispiel als Festkörpergelenke ausgebildet sind. Alternativ und abweichend von der Darstellung in Figur 10 können die Gelenke zwischen den 140, 150, 160, 170, 240, 250, 260, 270 als formschlüssige Gelenke beispielsweise mit Wellen oder Achszapfen und korrespondierenden Lagerschalen ausgebildet sein.

In den Bereichen der Gelenke zwischen benachbarten Gliedern 140, 150, 160, 170, 240, 250, 260, 270 sind die Glieder 140, 150, 160, 170, 240, 250, 260, 270 ferner jeweils durch Abstandsbauteile 350, 360, 370 miteinander verbunden. Je ein Ende 351, 361, 371 jedes Abstandsbauteils 350, 360, 370 ist durch ein Gelenk mit dem Übergangsbereich zwischen zwei benachbarten Gliedern 140, 150, 160, 170 an der ersten Seite 34 des Spatels 30 verbunden, und ein zweites Ende 352, 362, 372 jedes Abstandsbauteils 350, 360, 370 ist über ein Gelenk mit dem Übergangsbereich zwischen zwei benachbarten Gliedern 240, 250, 260, 270 an der zweiten Seite des Spatels 30 verbunden. Auch die Gelenke an den Enden 351, 352, 361, 362, 371, 372 der Abstandsbauteile 350, 360, 370 sind bei dem dargestellten Beispiel als Festkörpergelenke ausgebildet, können jedoch abweichend von der Darstellung in Figur 10 beispielsweise als formschlüssige Gelenke mit Wellen oder Achszapfen in korrespondierenden Lagerschalen ausgebildet sein.

Bei starrer Ausgestaltung der äußerst distalen Glieder 170, 270 können abweichend von der Darstellung in Figur 10 die Gelenke zwischen diesen und dem äußerst distalen Abstandsbauteil 370 entfallen.

In jedem Abstandsbauteil 350, 360, 370 ist eine Führungseinrichtung 358, 368, 378 in Gestalt einer Öffnung oder Öse, in der die Kraftübertragungseinrichtung 80 angeordnet und geführt ist, vorgesehen. Auch an dem distalen Ende des proximalen Abschnitts 40 des Spatels 30 ist eine Führungseinrichtung 48 vorgesehen. Bei dem dargestellten Beispiel ist bei jedem Abstandsbauteil 350, 360, 370 das Verhältnis des Abstands der Führungseinrichtung 358, 368, 378 von dem ersten Ende 351, 361, 371 des Abstandsbauteils 350, 360, 370 an der ersten Seite 34 des Spatels 30 und des Abstands der Führungseinrichtung 358, 368, 378 von dem zweiten Ende 352, 362, 372 des Abstandsbauteils 350, 360, 370 an der zweiten Seite 36 des Spatels 30 unterschiedlich und verschieden von dem Verhältnis der Abstände der Führungseinrichtung 48 an dem distalen Ende des proximalen Abschnitts 40 des Spatels 30 von der ersten Seite 34 und von der zweiten Seite 36 des Spatels 30. Diese unterschiedlichen Verhältnisse der Abstände bewirken, dass durch Schwenken des Griffteils 20 und die damit hervorgerufene Bewegung der Kraftübertragungseinrichtung 80 eine Verformung des Spatels 30 bewirkt werden kann.

Bei allen anhand der Figuren 1 bis 10 dargestellten Laryngoskopen 10 kann die Anzahl der Glieder 40, 50, 60, 70, 140, 150, 160, 170, 240, 250, 260, 270 von den Darstellungen in den Figuren abweichen. Abweichend von den Darstellungen in den Figuren 7 bis 10 können bei diesen Laryngoskopen die Kraftübertragungseinrichtungen zur Übertragung von Druck- oder Schubkräften zur Vergrößerung der Krümmung des Spatels 30 ausgebildet sein. Dazu sind insbesondere bei den anhand der Figuren 7 bis 9 dargestellten Laryngoskopen 10 die Gelenke 46, 56, 66 zwischen den Gliedern 40, 50, 60, 70 abweichend von den Darstellungen in den Figuren 7 bis 9 jeweils nicht an der zweiten Seite 36, sondern an der ersten Seite 34 des Spatels 30 angeordnet, ähnlich wie bei dem anhand der Figuren 5 und 6 dargestellten Laryngoskop.

### Bezugszeichen

- **10**: **Laryngoskop**
- **20**: **Griffteil** des Laryngoskops 10
- 26: Gelenk zwischen dem Griffteil 20 und dem proximalen Ende 32 des Spatels 30
- **30**: **Spatel** des Laryngoskops 10
- 32: proximales Ende des Spatels 30
- 34: zur Anlage an der Zunge und am Zungengrund eines Patienten vorgesehene Seite des Spatels 30
- 36: dem Gaumen eines Patienten zuzuwendende Seite des Spatels 30
- 38: distales Ende des Spatels 30
- **40**: **erstes Glied** des Spatels 30 oder proximaler Abschnitt des Spatels 30
- 42: proximales Ende des ersten Glieds 40
- 44: distales Ende des ersten Glieds 40
- 46: Gelenk zwischen dem ersten Glied 40 und dem zweiten Glied 50
- 48: Führungseinrichtung an dem ersten Glied oder dem proximalen Abschnitt 40 zum Führen der Kraftübertragungseinrichtung 80
- **50**: **zweites Glied** des Spatels 30
- 52: proximales Ende des zweiten Glieds 50
- 54: distales Ende des zweiten Glieds 50
- 56: Gelenk zwischen dem zweiten Glied 50 und dem dritten Glied 60
- 58: Führungseinrichtung an dem zweiten Glied 50 zum Führen der Kraftübertragungseinrichtung 80
- **60**: **drittes Glied** des Spatels 30
- 62: proximales Ende des dritten Glieds 60
- 64: distales Ende des dritten Glieds 60
- 66: Gelenk zwischen dem dritten Glied 60 und dem vierten Glied 70
- 68: Führungseinrichtung an dem dritten Glied 60 zum Führen der Kraftübertragungseinrichtung 80
- **70**: **viertes Glied** des Spatels 30
- 72: proximales Ende des vierten Glieds 70
- 74: distales Ende des vierten Glieds 70
- **80**: **Kraftübertragungseinrichtung** des Spatels 20
- 82: proximales Ende der Kraftübertragungseinrichtung 80
- 84: Umlenkeinrichtung in dem ersten Glied 40
- 85: Festkörpergelenk zwischen der Umlenkeinrichtung 84 und dem ersten Glied 40
- 87: distales Ende der Kraftübertragungseinrichtung 80
- 91: erster Hebel
- 92: zweiter Hebel
- 93: dritter Hebel
- 94: erste Zugstange
- 95: zweite Zugstange
- 96: dritte Zugstange
- 97: vierte Zugstange
- 140: erstes Glied einer ersten Kette von Gliedern
- 150: zweites Glied der ersten Kette von Gliedern
- 160: drittes Glied der ersten Kette von Gliedern
- 170: viertes Glied der ersten Kette von Gliedern
- 240: erstes Glied einer zweiten Kette von Gliedern
- 250: zweites Glied der zweiten Kette von Gliedern
- 260: drittes Glied der zweiten Kette von Gliedern
- 270: viertes Glied der zweiten Kette von Gliedern
- 350: erstes Abstandbauteil des Spatels 30
- 351: erstes Ende des ersten Abstandsbauteils 350
- 352: zweites Ende des ersten Abstandsbauteils 350
- 358: Führungseinrichtung in dem ersten Abstandsbauteil 350
- 360: zweites Abstandbauteil des Spatels 30
- 361: erstes Ende des zweiten Abstandsbauteils 360
- 362: zweites Ende des zweiten Abstandsbauteils 360
- 368: Führungseinrichtung in dem zweiten Abstandsbauteil 360
- 370: drittes Abstandbauteil des Spatels 30
- 371: erstes Ende des dritten Abstandsbauteils 370
- 372: zweites Ende des dritten Abstandsbauteils 370
- 378: Führungseinrichtung in dem dritten Abstandsbauteil 370

## Patentansprüche

1. **Spatel** (30) für ein adaptierbares oder adaptives Laryngoskop (10), mit:
einem **proximalen Ende** (32), das mit einem Griffteil (20) zum manuellen Halten des Laryngoskops (10) gelenkig verbunden oder verbindbar ist;
einem **distalen Ende** (38), zum Einführen in den Rachen eines Patienten;
einer bei vorgesehener Verwendung des Spatels (30) an der **Zunge** des Patienten anliegenden **ersten Seite** (34);
einer bei vorgesehener Verwendung des Spatels (30) dem **Gaumen** des Patienten zugewandten **zweiten Seite** (36),
einer Mehrzahl von in Längsrichtung des Spatels (30) hintereinander angeordneten und starren **Gliedern** (40, 50, 60, 70), die sich jeweils von der ersten Seite (34) des Spatels (30) bis zu der zweiten Seite (36) des Spatels (30) erstrecken;
einer Mehrzahl von **Gelenken** (46, 56, 66), wobei jedes der Mehrzahl von Gelenken (46, 56, 66) zwei benachbarte Glieder (40, 50, 60, 70) gelenkig verbindet;
einer **Kraftübertragungseinrichtung** (80) zum Übertragen einer Kraft, mit einem proximalen Ende (82) und einem distalen Ende (87),
wobei das distale Ende (87) der Kraftübertragungseinrichtung (80) mit dem distalen Ende (38) des Spatels (30) mechanisch verbunden ist,
wobei die Kraftübertragungseinrichtung (80) zwischen ihrem distalen Ende (87) und ihrem proximalen Ende (82) relativ zu Gliedern (40, 50, 60) des Spatels (30) in Längsrichtung der Kraftübertragungseinrichtung (80) bewegbar ist,
wobei das proximale Ende (82) der Kraftübertragungseinrichtung (80) mit dem Griffteil (20) derart mechanisch gekoppelt ist oder dafür vorgesehen und ausgebildet ist, mit dem Griffteil (20) derart mechanisch gekoppelt zu werden, dass durch Schwenken des Griffteils (20) relativ zu dem proximalen Ende (32) des Spatels (30) die Kraftübertragungseinrichtung (80) relativ zu dem proximalen Ende (32) des Spatels bewegbar ist
wobei, dass die Kraftübertragungseinrichtung (80) flexible ist **dadurch gekennzeichnet, dass** die Kraftübertragungseinrichtung (80) aus einem elastischen Material gebildet.

2. Spatel (30) nach dem vorangehenden Anspruch, bei dem
entweder die **Kraftübertragungseinrichtung** (80) nahe der **ersten Seite** (34) des Spatels (30) angeordnet und zur Übertragung einer **Zugkraft** ausgebildet ist, und eines oder mehrere oder alle der Mehrzahl von Gelenken (46, 56, 66) nahe der zweiten Seite (36) des Spatels (30) angeordnet sind,
oder eines oder mehrere oder alle der Mehrzahl von Gelenken (46, 56, 66) nahe der ersten Seite (34) des Spatels (30) angeordnet sind und die **Kraftübertragungseinrichtung** (80) nahe der **zweiten Seite** (36) des Spatels (30) angeordnet und zur Übertragung einer **Druckkraft** ausgebildet ist.

3. Spatel (30) nach einem der vorangehenden Ansprüche, bei dem eines oder mehrere oder alle der Mehrzahl von **Gliedern** (40, 50, 60, 70) bezogen auf Schnittebenen orthogonal zur Längsrichtung des Spatels (30) einen **ringförmigen** Querschnitt oder einen U-förmigen Querschnitt oder einen **C-förmigen** Querschnitt aufweisen.

4. Spatel (30) nach dem vorangehenden Anspruch, bei dem die **Kraftübertragungseinrichtung** (80) **innerhalb** eines von einem oder mehreren oder allen der Mehrzahl von Gliedern (40, 50, 60, 70) zumindest teilweise umschlossenen Hohlraums angeordnet ist.

5. Spatel (30) nach einem der vorangehenden Ansprüche, ferner mit: einer **Führungseinrichtung** (48, 58, 68) in einem der Mehrzahl von Gliedern (40, 50, 60), zum Führen der Kraftübertragungseinrichtung (80).

6. Spatel (30) nach einem der vorangehenden Ansprüche, bei dem die Führungseinrichtung (48, 58, 68) einen **Kanal** oder eine **Öse** umfasst.

7. Spatel (30) nach einem der vorangehenden Ansprüche, ferner mit:
einer **Umlenkeinrichtung** (84), an der die Kraftübertragungseinrichtung (80) anliegt oder mit der die Kraftübertragungseinrichtung (80) mechanisch verbunden ist oder die Teil der Kraftübertragungseinrichtung (80) ist, wobei die Kraftübertragungseinrichtung (80) distal der Umlenkeinrichtung (84) in einer ersten Richtung und proximal der Umlenkeinrichtung (84) in einer zweiten Richtung, die sich von der ersten Richtung unterscheidet, verläuft.

8. Spatel (30) nach einem der vorangehenden Ansprüche, bei dem die Kraftübertragungseinrichtung (80) eine **Übersetzungseinrichtung** (84, 91, 92, 93), die eine erste Kraft und einen ersten Weg proximal der Übersetzungseinrichtung (84, 91, 92, 93) in eine zweite Kraft und einen zweiten Weg distal der Übersetzungseinrichtung (84, 91, 92, 93) übersetzt, aufweist.

9. **Spatel** (30) für ein adaptives Laryngoskop (10), mit:
einem **proximalen Ende** (32), das mit einem Griffteil (20) zum manuellen Halten des Laryngoskops (10) verbunden oder verbindbar ist;
einem **distalen Ende** (38), zum Einführen in den Rachen eines Patienten;
einer ersten Kette aus einer Mehrzahl von in Längsrichtung des Spatels (30) hintereinander angeordneten und jeweils paarweise gelenkig verbundenen **Gliedern** (140, 150, 160, 170), die sich von dem proximalen Ende (32) bis zu dem distalen Ende (38) des Spatels (30) erstreckt;
einer zweiten Kette aus einer Mehrzahl von in Längsrichtung des Spatels (30) hintereinander angeordneten und jeweils paarweise gelenkig verbundenen **Gliedern** (240, 250, 260, 270), die sich von dem proximalen Ende (32) bis zu dem distalen Ende (38) des Spatels (30) erstreckt;
einer Mehrzahl von Abstandsbauteilen (350, 360), die jeweils einen Punkt an einem Glied (150, 160) der ersten Kette mit einem Punkt an einem Glied (250, 260) der zweiten Kette gelenkig und mit einem vorbestimmten Abstand verbinden,
**dadurch gekennzeichnet, dass** der Spatel ausgebildet ist mit
einer **Kraftübertragungseinrichtung** (80) zum Übertragen einer Kraft, mit einem proximalen Ende (82) und einem distalen Ende (87),
wobei das distale Ende (87) der Kraftübertragungseinrichtung (80) mit dem distalen Ende (38) des Spatels (30) mechanisch verbunden ist,
wobei die Kraftübertragungseinrichtung (80) zwischen ihrem distalen Ende (87) und ihrem proximalen Ende (82) relativ zu Gliedern (40, 50, 60, 70) des Spatels (30) in Längsrichtung der Kraftübertragungseinrichtung (80) bewegbar ist
wobei die Kraftübertragungseinrichtung (80) flexible ist, wobei die Kraftübertragungseinrichtung aus einem elastischen Material gebildet ist.

10. Spatel (30) nach den vorangehenden Ansprüchen, ferner mit:
einer Führungseinrichtung (358, 368) an einem der Mehrzahl von Abstandsbauteilen (350, 360), zum Führen der Kraftübertragungseinrichtung (80).

11. Spatel (30) nach dem vorangehenden Ansprüche, bei dem
an einem ersten Abstandsbauteil (350) der Mehrzahl von Abstandsbauteilen (350, 360) eine erste Führungseinrichtung (358) zum Führen der Kraftübertragungseinrichtung (80) vorgesehen ist,
an einem zweiten Abstandsbauteil (360) der Mehrzahl von Abstandsbauteilen (350, 360) eine zweite Führungseinrichtung (368) zum Führen der Kraftübertragungseinrichtung (80) vorgesehen ist,
ein ersten Verhältnis zwischen dem Abstand der ersten Führungseinrichtung (358) von einem ersten Ende (351) des ersten Abstandsbauteils (350), das dem zugeordneten Glied (150) der ersten Kette zugewandt ist, und dem Abstand der ersten Führungseinrichtung (358) von einem zweiten Ende (352) des ersten Abstandsbauteils (350), das dem zugeordneten Glied (250) der zweiten Kette zugewandt ist, verschieden ist von einem zweiten Verhältnis zwischen dem Abstand der zweiten Führungseinrichtung (368) von einem ersten Ende (361) des zweiten Abstandsbauteils (360), das dem zugeordneten Glied (160) der ersten Kette zugewandt ist, und dem Abstand der zweiten Führungseinrichtung (368) von einem zweiten Ende (362) des zweiten Abstandsbauteils (360), das dem zugeordneten Glied (260) der zweiten Kette zugewandt ist.

12. **Laryngoskop** (10) mit:
einem **Spatel** (30) nach einem der vorangehenden Ansprüche;
einem **Griffteil** (20), der mit dem proximalen Ende (32) des Spatels (30) verbunden oder verbindbar ist.

13. Laryngoskop (10) nach dem vorangehenden Anspruch, bei dem
das proximalen Ende (32) des **Spatels** (30) **gelenkig mit dem Griffteil** (20) verbunden oder verbindbar ist,
das proximale Ende (82) der **Kraftübertragungseinrichtung** (80) derart **mit dem Griffteil** (20) gekoppelt oder koppelbar ist, dass durch Schwenken des Griffteils (20) relativ zu dem proximalen Ende (32) des Spatels (30) eine Zugkraft oder eine Druck- oder Schubkraft auf die Kraftübertragungseinrichtung (80) ausgeübt werden kann.

## Claims

1. A **blade** (30) for an adaptable or adaptive laryngoscope (10), comprising:
a **proximal end** (32), which is articulatedly connected or connectable to a handle part (20) for manually holding the laryngoscope (10);
a **distal end** (38) for insertion into the throat of a patient;
a **first side** (34) resting on the **tongue** of the patient during the intended use of the blade (30);
a **second side** (36) facing the **palate** of the patient during the intended use of the blade (30),
a plurality of rigid **members** (40, 50, 60, 70) which are arranged one after another in the longitudinal direction of the blade (30) and each of which extend from the first side (34) of the blade (30) to the second side (36) of the blade (30);
a plurality of **joints** (46, 56, 66), wherein each of the plurality of joints (46, 56, 66) articulatedly connects two adjacent members (40, 50, 60, 70);
a **force transmitting mechanism** (80) for transmitting a force, comprising a proximal end (82) and a distal end (87),
wherein the distal end (87) of the force transmitting mechanism (80) is mechanically connected to the distal end (38) of the blade (30),
wherein the force transmitting mechanism (80) can be moved between its distal end (87) and its proximal end (82) relative to members (40, 50, 60) of the blade (30) in the longitudinal direction of the force transmitting mechanism (80),
wherein the proximal end (82) of the force transmitting mechanism (80) is mechanically coupled to the handle part (20) or is provided and designed to be mechanically coupled to the handle part (20) in such manner that the force transmitting mechanism (80) can be moved relative to the proximal end (32) of the blade (30) by pivoting the handle part (20) relative to the proximal end (32) of the blade,
wherein the force transmitting mechanism (80) is flexible, **characterised in that** the force transmitting mechanism (80) is formed from an elastic material.

2. The blade (30) according to the preceding claim, in which
either the **force transmitting mechanism** (80) is arranged near the **first side** (34) of the blade (30) and is designed to transmit a **tensile force,** and one or a plurality or all of the plurality of joints (46, 56, 66) are arranged near the second side (36) of the blade (30),
or one or a plurality or all of the plurality of joints (46, 56, 66) are arranged near the first side (34) of the blade (30) and the **force transmitting mechanism** (80) is arranged near the **second side** (36) of the blade (30) and is designed to transmit a **pressing force.**

3. The blade (30) according to one of the preceding claims, in which one or a plurality or all of the plurality of **members** (40, 50, 60, 70) have an **annular** cross-section or a U-shaped cross-section or a **C-shaped** cross-section in relation to section planes orthogonal to the longitudinal direction of the blade (30).

4. The blade (30) according to the preceding claim, in which the **force transmitting mechanism** (80) is arranged **within** a cavity at least partially enclosed by one or a plurality or all of the plurality of members (40, 50, 60, 70).

5. The blade (30) according to one of the preceding claims, further comprising: a **guide mechanism** (48, 58, 68) in one of the plurality of members (40, 50, 60), for guiding the force transmitting mechanism (80).

6. The blade (30) according to one of the preceding claims, in which the guide mechanism (48, 58, 68) comprises a **channel** or an **eyelet.**

7. The blade (30) according to one of the preceding claims, further comprising:
a **deflecting mechanism** (84) on which the force transmitting mechanism (80) rests or to which the force transmitting mechanism (80) is mechanically connected or which is part of the force transmitting mechanism (80), wherein the force transmitting mechanism (80) extends distally from the deflecting mechanism (84) in a first direction and proximally from the deflecting mechanism (84) in a second direction which differs from the first direction.

8. The blade (30) according to one of the preceding claims, in which the force transmitting mechanism (80) has a **converting mechanism** (84, 91, 92, 93) that converts a first force and a first path proximal from the converting mechanism (84, 91, 92, 93) into a second force and a second path distal from the converting mechanism (84, 91, 92, 93).

9. The **blade** (30) for an adaptive laryngoscope (10), comprising:
a **proximal end** (32), which is connected or connectable to a handle part (20) for manually holding the laryngoscope (10);
a **distal end** (38) for insertion into the throat of a patient;
a first chain of a plurality of **members** (140, 150, 160, 170), which are arranged one after another in the longitudinal direction of the blade (30) and which are each articulatedly connected in pairs, which chain extends from the proximal end (32) to the distal end (38) of the blade (30);
a second chain of a plurality of **members** (240, 250, 260, 270), which are arranged one after another in the longitudinal direction of the blade (30) and which are each articulatedly connected in pairs, which chain extends from the proximal end (32) to the distal end (38) of the blade (30);
a plurality of spacer components (350, 360), each of which articulatedly connects a point on a member (150, 160) of the first chain to a point on a member (250, 260) of the second chain at a predetermined spacing, **characterised in that** the blade is formed with
a **force transmitting mechanism** (80) for transmitting a force, comprising a proximal end (82) and a distal end (87),
wherein the distal end (87) of the force transmitting mechanism (80) is mechanically connected to the distal end (38) of the blade (30),
wherein the force transmitting mechanism (80) can be moved between its distal end (87) and its proximal end (82) relative to members (40, 50, 60, 70) of the blade (30) in the longitudinal direction of the force transmitting mechanism (80),
wherein the force transmitting mechanism (80) is flexible, wherein the force transmitting mechanism is formed from an elastic material.

10. The blade (30) according to the preceding claims, further comprising:
a guide mechanism (358, 368) on one of the plurality of spacer components (350, 360), for guiding the force transmitting mechanism (80).

11. The blade (30) according to the preceding claims, in which
a first guide mechanism (358) for guiding the force transmitting mechanism (80) is provided on a first spacer component (350) of the plurality of spacer components (350, 360),
a second guide mechanism (368) for guiding the force transmitting mechanism (80) is provided on a second spacer component (360) of the plurality of spacer components (350, 360),
a first ratio between the spacing of the first guide mechanism (358) from a first end (351) of the first spacer component (350), which faces the assigned member (150) of the first chain, and the spacing of the first guide mechanism (358) from a second end (352) of the first spacer component (350), which faces the assigned member (250) of the second chain, is different from a second ratio between the spacing of the second guide mechanism (368) from a first end (361) of the second spacer component (360), which faces the assigned member (160) of the first chain, and the spacing of the second guide mechanism (368) from a second end (362) of the second spacer component (360), which faces the assigned member (260) of the second chain.

12. A **laryngoscope** (10) comprising:
a **blade** (30) according to one of the preceding claims;
a **handle part** (20), which is connected or connectable to the proximal end (32) of the blade (30).

13. The laryngoscope (10) according to the preceding claim, in which
the proximal end (32) of the **blade** (30) is **articulatedly** connected or connectable **to the handle part** (20),
the proximal end (82) of the **force transmitting mechanism** (80) is coupled or couplable **to the handle part** (20) in such manner that a tensile force or a pressing or shear force can be exerted on the force transmitting mechanism (80) by pivoting the handle part (20) relative to the proximal end (32) of the blade (30).

## Revendications

1. **Lame** (30) pour un laryngoscope adaptable ou adaptatif (10), comportant :
une **extrémité proximale** (32), qui est reliée ou peut être reliée de manière articulée à une partie de préhension (20) pour tenir manuellement le laryngoscope (10) ;
une extrémité **distale** (38), à insérer dans la gorge d'un patient ;
un **premier côté** (34) s'appliquant contre la **langue** du patient lors d'une utilisation prévue de la lame (30) ;
un **second côté** (36) tourné vers le **palais** du patient lors d'une utilisation prévue de la lame (30),
une pluralité de **maillons** (40, 50, 60, 70) agencés les uns derrière les autres dans la direction longitudinale de la lame (30) et rigides, qui s'étendent respectivement depuis le premier côté (34) de la lame (30) jusqu'au second côté (36) de la lame (30) ;
une pluralité d'**articulations** (46, 56, 66), dans laquelle chacune de la pluralité d'articulations (46, 56, 66) relie de manière articulée deux maillons (40, 50, 60, 70) adjacents ;
un **dispositif de transmission de force** (80) pour transmettre une force, comportant une extrémité proximale (82) et une extrémité distale (87),
dans laquelle l'extrémité distale (87) du dispositif de transmission de force (80) est reliée mécaniquement à l'extrémité distale (38) de la lame (30),
dans laquelle le dispositif de transmission de force (80) est mobile entre son extrémité distale (87) et son extrémité proximale (82) par rapport à des maillons (40, 50, 60) de la lame (30) dans la direction longitudinale du dispositif de transmission de force (80),
dans laquelle l'extrémité proximale (82) du dispositif de transmission de force (80) est accouplée mécaniquement à la partie de préhension (20) ou est prévue et conçue pour être accouplée mécaniquement à la partie de préhension (20) de telle sorte qu'en faisant pivoter la partie de préhension (20) par rapport à l'extrémité proximale (32) de la lame (30), le dispositif de transmission de force (80) est mobile par rapport à l'extrémité proximale (32) de la lame
dans laquelle le dispositif de transmission de force (80) est flexible, **caractérisée en ce que** le dispositif de transmission de force (80) est formé d'un matériau élastique.

2. Lame (30) selon la revendication précédente, dans laquelle
soit le **dispositif de transmission de force** (80) est agencé à proximité du **premier côté** (34) de la lame (30) et est conçu pour transmettre une **force de traction,** et un ou plusieurs ou la totalité de la pluralité d'articulations (46, 56, 66) sont agencées à proximité du second côté (36) de la lame (30),
soit une ou plusieurs ou la totalité de la pluralité d'articulations (46, 56, 66) sont agencées à proximité du premier côté (34) de la lame (30) et le **dispositif de transmission de force** (80) est agencé à proximité du **second côté** (36) de la lame (30) et est conçu pour transmettre une **force de pression.**

3. Lame (30) selon l'une des revendications précédentes, dans laquelle un ou plusieurs ou la totalité de la pluralité de **maillons** (40, 50, 60, 70) présentent, par rapport à des plans de coupe orthogonaux à la direction longitudinale de la lame (30), une section transversale **annulaire** ou une section transversale en forme de U ou une section transversale **en forme de C.**

4. Lame (30) selon la revendication précédente, dans laquelle le **dispositif de transmission de force** (80) est agencé **à l'intérieur** d'une cavité au moins partiellement entourée par un ou plusieurs ou la totalité de la pluralité de maillons (40, 50, 60, 70).

5. Lame (30) selon l'une des revendications précédentes, comportant en outre : un **dispositif de guidage** (48, 58, 68) dans l'un de la pluralité de maillons (40, 50, 60), pour guider le dispositif de transmission de force (80).

6. Lame (30) selon l'une des revendications précédentes, dans laquelle le dispositif de guidage (48, 58, 68) comprend un **canal** ou un **œillet.**

7. Lame (30) selon l'une des revendications précédentes, comportant en outre :
un **dispositif de renvoi** (84), contre lequel le dispositif de transmission de force (80) s'applique ou auquel le dispositif de transmission de force (80) est relié mécaniquement ou qui fait partie du dispositif de transmission de force (80), dans laquelle le dispositif de transmission de force (80) s'étend de manière distale par rapport au dispositif de renvoi (84) dans une première direction et de manière proximale par rapport au dispositif de renvoi (84) dans une seconde direction qui est différente de la première direction.

8. Lame (30) selon l'une des revendications précédentes, dans laquelle le dispositif de transmission de force (80) comprend un **dispositif de traduction** (84, 91, 92, 93) qui traduit une première force et un premier trajet proximal par rapport au dispositif de traduction (84, 91, 92, 93) en une seconde force et un second trajet distal par rapport au dispositif de traduction (84, 91, 92, 93).

9. **Lame** (30) pour un laryngoscope adaptatif (10), comportant :
une **extrémité proximale** (32) qui est reliée ou peut être reliée à une partie de préhension (20) pour tenir manuellement le laryngoscope (10) ;
une extrémité **distale** (38), à insérer dans la gorge d'un patient ;
une première chaîne constituée d'une pluralité de **maillons** (140, 150, 160, 170) agencés les uns derrière les autres dans la direction longitudinale de la lame (30) et articulés deux à deux, qui s'étend de l'extrémité proximale (32) jusqu'à l'extrémité distale (38) de la lame (30) ;
une seconde chaîne constituée d'une pluralité de **maillons** (240, 250, 260, 270) agencés les uns derrière les autres dans la direction longitudinale de la lame (30) et articulés deux à deux, qui s'étend de l'extrémité proximale (32) jusqu'à l'extrémité distale (38) de la lame (30) ;
une pluralité d'éléments d'espacement (350, 360), qui relient respectivement de manière articulée et à une distance prédéterminée un point sur un maillon (150, 160) de la première chaîne à un point sur un maillon (250, 260) de la seconde chaîne, **caractérisée en ce que** la lame est conçue avec
un **dispositif de transmission de force** (80) pour transmettre une force, comportant une extrémité proximale (82) et une extrémité distale (87),
dans laquelle l'extrémité distale (87) du dispositif de transmission de force (80) est reliée mécaniquement à l'extrémité distale (38) de la lame (30),
dans laquelle le dispositif de transmission de force (80) est mobile entre son extrémité distale (87) et son extrémité proximale (82) par rapport à des maillons (40, 50, 60, 70) de la lame (30) dans la direction longitudinale du dispositif de transmission de force (80)
dans laquelle le dispositif de transmission de force (80) est flexible, dans laquelle le dispositif de transmission de force est formé d'un matériau élastique.

10. Lame (30) selon les revendications précédentes, comportant en outre :
un dispositif de guidage (358, 368) sur l'un de la pluralité d'éléments d'espacement (350, 360), pour guider le dispositif de transmission de force (80).

11. Lame (30) selon les revendications précédentes, dans laquelle
un premier dispositif de guidage (358) est prévu sur un premier élément d'espacement (350) de la pluralité d'éléments d'espacement (350, 360) pour guider le dispositif de transmission de force (80),
un second dispositif de guidage (368) est prévu sur un second élément d'espacement (360) de la pluralité d'éléments d'espacement (350, 360) pour guider le dispositif de transmission de force (80),
un premier rapport entre la distance du premier dispositif de guidage (358) à une première extrémité (351) du premier élément d'espacement (350), qui fait face au maillon (150) associé de la première chaîne, et la distance du premier dispositif de guidage (358) à une seconde extrémité (352) du premier élément d'espacement (350), qui fait face au maillon (250) associé de la seconde chaîne, est différente d'un second rapport entre la distance du second dispositif de guidage (368) à une première extrémité (361) du second élément d'espacement (360), qui fait face au maillon (160) associé de la première chaîne, et la distance du second dispositif de guidage (368) à une seconde extrémité (362) du second élément d'espacement (360), qui fait face au maillon (260) associé de la seconde chaîne.

12. **Laryngoscope** (10) comportant :
une **lame** (30) selon l'une des revendications précédentes ;
une **partie de préhension** (20), qui est reliée ou peut être reliée à l'extrémité proximale (32) de la lame (30).

13. Laryngoscope (10) selon la revendication précédente, dans lequel
l'extrémité proximale (32) de la **lame** (30) est reliée ou peut être reliée **de manière articulée à la partie de préhension** (20),
l'extrémité proximale (82) **du dispositif de transmission de force** (80) est accouplée ou peut être accouplée à **la partie de préhension** (20) de telle sorte qu'en faisant pivoter la partie de préhension (20) par rapport à l'extrémité proximale (32) de la lame (30), une force de traction ou une force de pression ou de poussée peut être exercée sur le dispositif de transmission de force (80).
